(19) Europäisches Patentamt
European Patent Office
Office européen des brevets

(11) EP 0 548 790 B1

(12) EUROPÄISCHE PATENTSCHRIFT

(45) Veröffentlichungstag und Bekanntmachung des Hinweises auf die Patenterteilung:
**01.05.1996 Patentblatt 1996/18**

(51) Int. Cl.[6]: **C07D 498/04**, A61K 31/42

(21) Anmeldenummer: **92121455.7**

(22) Anmeldetag: **17.12.1992**

# (54) (1'S)-Hydroxyalkyloxapenem-3-carbonsäuren und ihre Verwendung als Betalactamasehemmer

(1'S)-Hydroxyalkyloxapenem-3-carboxylic acids and their use as beta-lactamase inhibitors

Acides (1'S)-hydroxyalkyloxapenem-3-carboxyliques et leur utilisation comme inhibiteurs de la bêta-lactamase

(84) Benannte Vertragsstaaten:
**AT BE CH DE DK ES FR GB IT LI NL**

(30) Priorität: **20.12.1991 DE 4142423**

(43) Veröffentlichungstag der Anmeldung:
**30.06.1993 Patentblatt 1993/26**

(73) Patentinhaber: **ELI LILLY AND COMPANY**
**Indianapolis, Indiana 46285 (US)**

(72) Erfinder: **Pfaendler, Hans Rudolf, Prof. Dr.**
**81475 München (DE)**

(74) Vertreter: **Hudson, Christopher Mark et al**
**Lilly Industries Limited**
**European Patent Operations**
**Erl Wood Manor**
**Windlesham Surrey GU20 6PH (GB)**

(56) Entgegenhaltungen:
**EP-A- 0 301 394** **EP-A- 0 362 622**
**EP-A- 0 474 038**

- **CHEMICAL ABSTRACTS, vol. 114, no. 9, 4. M rz 1991, Columbus, Ohio, US; abstract no. 81343m, M. MURAKAMI ET AL.**

Bemerkungen:
Die Akte enthält technische Angaben, die nach dem Eingang der Anmeldung eingereicht wurden und die nicht in dieser Patentschrift enthalten sind.

EP 0 548 790 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Rank Xerox (UK) Business Services
2.11.3/3.4

## Beschreibung

Die Erfindung betrifft 1-Oxapenem-3-carbonsäuren der folgenden Struktur:

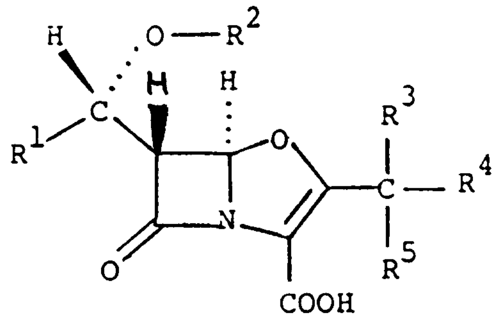

in der $R^1$, $R^3$, $R^4$ und $R^5$ unabhängig voneinander pharmazeutisch annehmbare Gruppen, die via Kohlenstoff-Kohlenstoff-Einfachbindungen mit dem übrigen Molekülteil verbunden sind und die ein bis 10 Kohlenstoffatome aufweisen, und in der $R^2$ = H, Alkyl oder Acyl mit bis zu 10 Kohlenstoffatomen oder einen anorganischen Säurerest bedeuten. Diese Verbindungen, wie auch ihre pharmazeutisch annehmbaren Salze, Ester und Amidderivate sind besonders wertvolle Betalactamasehemmer. Gegenstand der Erfindung sind weiterhin diese Verbindungen enthaltende pharmazeutische Zubereitungen und Behandlungsverfahren, sowie Verfahren zur Herstellung solcher Verbindungen.

Die Erfindung betrifft 2-substituierte Oxapenem-3-carbonsäuren, die in 2-Stellung mit besonderen Resten versehen sind, die bereits in EP 89117433.6 beschrieben worden sind und die dem Oxapenem-Nucleus die für die Verwendung als Betalactamasehemmer notwendige Stabilität verleihen. In der 6-Stellung sind diese Verbindungen monosubstituiert und enthalten einen (1S)-Hydroxyalkylrest, der am Sauerstoff oder an einem Kohlenstoffatom weiter substituiert sein kann. Diese optisch aktiven Verbindungen sind wertvolle Betalactamasehemmer und sie können durch die allgemeine Strukturformel

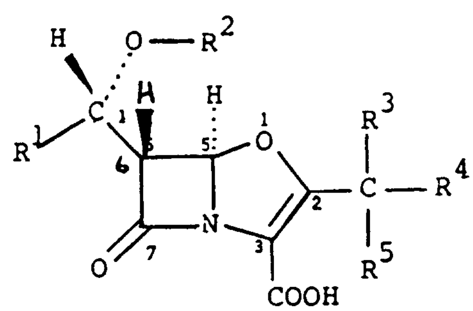

dargestellt werden, in der $R^1$, $R^3$, $R^4$ und $R^5$ unabhängig von einander ausgewählt werden aus den pharmazeutisch annehmbaren, über C-C-Einfachbindungen an den übrigen Molekülteil gebundenen Gruppen, die enthalten: substituiertes oder unsubstituiertes Alkyl, Alkenyl, Alkinyl, Cycloalkyl, Alkylcycloalkyl, Alkylcycloalkenyl, Cycloalkylalkyl, Alkenylcycloalkyl, Cycloalkenylalkyl, Aryl, Aralkyl, Aralkenyl, Aralkinyl, Carboxy oder Cyano, worin die vorhergehenden Alkyl, Alkenyl oder Alkinyl-Moleküle 1 bis 6 Kohlenstoffatome, die Cycloalkyl- oder die Cycloalkenyl-Molekülteile 3 bis 6 und die Aryl-Molekülteile 6 bis 10 Kohlenstoffatome enthalten, aromatisches oder aliphatisches Heterocyclyl, Heterocyclylalkyl, Heterocyclylalkenyl, Heterocyclylalkinyl, Alkylheterocyclyl, worin die vorhergehenden Alkyl-, Alkenyl- oder die Alkinyl-Molekülteile 1 bis 6 Kohlenstoffatome enthalten und der heterocyclische Molekülteil mono- oder bicyclisch ist und 3 bis 10 Ringatome enthält, wovon eines oder mehrere ausgewählt werden aus der Gruppe: Sauerstoff, Schwefel und Stickstoff, und wobei die Substituenten der oben aufgeführten Gruppen sein können: Geschütztes oder ungeschütztes Hydroxy, Hydroxyalkyl, Aminoalkyloxy, Alkyloxy, Acyloxy, Arylox, Heterocyclyloxy, Carbamoyl, Carbamoyloxy, Thiocarbamoyl, Thiocarbamoyloxy, Alkylcarbamoyloxy, Alkylthiocarbamoyloxy, Mercapto, Alkylthio, Hydroxyalkylthio, Aminoalkylthio, Amidinoalkylthio, Acylthio, Arylthio, Alkylheteroarylthio, Hydroxyalkylheteroarylthio, Heterocyclylthio, Carbamoylthio, Alkylcarbamoylthio, Thiocarbamoylthio, Alkylthiocarbamoylthio, geschütztes oder ungeschütztes Amino oder Monoalkylamino, Dialkylamino, Oxo, geschütztes oder ungeschütztes Oximino oder Alkylimino, Tetraalkylammonium, Cycloalkylamino, Arylamino, Heteroarylamino, Heterocyclylamino, Acylamino, Amidino, Alkylamidino, Guanidino, Alkylguanidino, Carbamoylamino, Alkylcarbamoylamino, Thiocarbamoylamino, Alkylthiocarbamoylamino, Nitro, Chlor, Brom, Fluor, Iod, Azido, Cyano, Alkylsulfinyl, Alkylsulfonyl, Sulfonamido, Sulfamoyloxy, Alkylsulfonyloxy oder geschütztes oder ungeschütztes Sulfo, Sulfoxy oder Carboxy, wobei die Substituenten unabhängig voneinander ein- oder mehrfach

auftreten und ihr Alkyl-Molekülteil 1 bis 6 Kohlenstoffatome, ihr Aryl-Molekülteil 6 bis 10 Kohlenstoffatome enthält, und wobei der heteroaromatische oder heterocyclische Molekülteil mono- oder bicyclisch ist und 3 bis 10 Ringatome enthält, wovon eines oder mehrere ausgewählt werden aus der Gruppe: Sauerstoff, Schwefel und Stickstoff, und in der $R^2$ Wasserstoff, Alkyl-oder Acyl bis zu 10 Kohlenstoffatomen, Sulfo, Aminosulfonyl oder Phosphoroyl bedeutet.

Die Schutzgruppen der oben erwähnten, geschützten Substituenten sind an sich bekannte, leicht entfernbare Reste, wie sie üblicherweise in der organischen Synthese zu diesem Zweck Verwendung finden. Solche Schutzgruppen finden sich beispielsweise in T. W. Greene, "Protective Groups in Organic Synthesis", Wiley, New York, 1981.

Weiterhin können zwei der Gruppen $R^3$, $R^4$ oder $R^5$ über Kohlenstoff, Sauerstoff, Stickstoff und Schwefel enthaltende Molekülteile miteinander verbrückt sein; sie sind dann Bestandteil eines carbocyclischen oder heterocyclischen Ringes, der drei-, vier-, fünf- oder sechsgliedrig sein kann.

Weiterhin können die beiden Gruppen $R^1$ und $R^2$ über Kohlenstoff, Sauerstoff, Stickstoff und Schwefel enthaltende Molekülteile miteinander verbrückt sein; sie sind dann Bestandteil eines drei-, vier-, fünf- oder sechsgliedrigen heterocyclischen Ringes.

Beispiele für verbrückende Molekülteile für $R^1$ und $R^2$ bzw. für $R^3$ und $R^4$ sind Methylen, Dimethylen, Trimethylen, Tetramethylen, Oxamethylen, Oxadimethylen, Dioxamethylen, Azadimethylen, Diazamethylen o.ä.

Pharmazeutisch annehmbare Gruppen $R^1$, $R^3$, $R^4$ und $R^5$, die über C-C-Einfachbindungen gebunden sind, sind Gruppen, wie sie beispielsweise bei den β-Lactamantibiotika üblich sind. Solche Gruppen findet man z. B. in M.L. Sassiver, A. Lewis in "Advances in Applied Microbiology, ed. D. Perlman, Academic Press, N. Y. (1970).

Die Erfindung betrifft weiterhin die pharmazeutisch annehmbaren Salze, Ester und Amidderivate der erfindungsgemässen Verbindungen.

Pharmazeutisch annehmbare Ester und Amidderivate sind seit langem bekannt und dienen der besseren oralen Verfügbarkeit vieler bekannter Antibiotika. Viele solcher Derivate, z. B. Pivaloyloxymethylester sind sogenannte Prodrugs und sind in W. Dürckheimer et al. in Adv. Drug Res. 17, 197 - 203 (1988) beschrieben.

Die Erfindung betrifft weiterhin Verfahren zur Herstellung solcher Verbindungen, solche Verbindungen enthaltende pharmazeutische Zubereitungen und Verfahren zur Behandlung, bei denen diese Verbindungen und Zubereitungen verabreicht werden, wenn eine antibiotische Wirkung indiziert ist.

Es besteht ein kontinuierlicher Bedarf an neuen Antibiotika. Unglücklicherweise gibt es bei irgendeinem gegebenen Antibiotikum keine statische Wirksamkeit, da bei der kontinuierlichen Verwendung im grossen Massstab selektiv resistente Stämme von pathogenen Bakterien neu gebildet werden. Dementsprechend geht die Suche nach neuen Antibiotika weiter.

Die Betalactamantibiotika haben mit rund zwei Drittel den bedeutendsten Anteil am Antibiotikamarkt. Sie sind besonders geschätzt wegen ihrer guten Verträglichkeit.

In den letzten Jahren sind jedoch in vielen Spitälern vermehrt schwere Infektionskrankheiten aufgetreten, die von stark Betalactamase-bildenden Keimen verursacht wurden und die selbst die relativ Betalactamase-unempfindlichen Cephalosporine der dritten Generation angreifen. Solche Infektionskrankheiten können durch diese Antibiotika allein nicht mehr bekämpft werden.

Eine Lösung zu diesem Problem bieten die Betalactamasehemmer. Sie werden zusammen mit einem Betalactamantibiotikum verabreicht und binden die bakteriellen Enzyme rasch und irreversibel. Das auf diese Weise geschützte Antibiotikum kann dann seine volle Wirkung entfalten.

Eine neuere Zusammenfassung über Resistenz-Enzyme und die Anwendung von Betalactamase-Inhibitoren findet sich in (H.C. Neu, The Amer. J. of Med. 79 (suppl. 5B), 1 (1985).

Stabile Oxapenem-carbonsäuren als Betalactamasehemmer sind in EP 89117433.6 beschrieben worden. Stoffe mit 6-Hydroxyalkylgruppen erwiesen sich dabei als gute Hemmer.

2-tert-Butyl-6-hydroxymethyloxapenem-3-carbonsäure (K-Salz) Ia erwies sich als wirksamer als Clavulansäure in der Kombination mit Mezlocillin gegen Penicillin resistenten Staph. aureus und Pseudomonas aeruginosa.

Die in der 6-Position eingebaute Hydroxymethylgruppe besitzt kein zusätzliches Asymmetriezentrum. Erst höhere homologe Seitenketten wie z. B. Hydroxyethyl beinhalten ein solches Zentrum. Die Untersuchung verschiedener stereoisomerer 2-tert-Butyl-6-hydroxyethyloxapenem-3-carbonsäuren (K-Salze) Ib - Ie zeigte eine überraschende Abhängigkeit der Betalactamase-Hemmung von der Konfiguration der asymmetrischen Hydroxyethyl-Seitenkette.

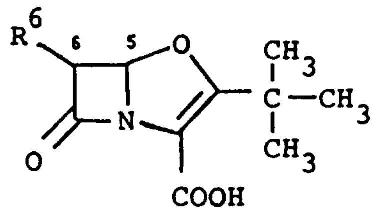

I

| | $R^6$ | Konfiguration | Hemmhofdurchmesser mm (E. coli TEM I) | |
|---|---|---|---|---|
| | | | I (10 mcg) | I + Cefaclor (10 + 30 mcg) |
| Ia | rac. Hydroxymethyl | trans (5R, 6R) | 17 | 22 |
| Ib | rac. Hydroxyethyl | trans (1'S, 5R, 6R) | 0 | 28 |
| Ic | rac. Hydroxyethyl (enthält ca. 20 % Ib) | cis (1'R, 5R, 6S) | 9 | 26 |
| Id | ((1'R)-Hydroxyethyl) | trans (5R, 6R) | 23 | 24 |
| Ie | ((1'S)-Hydroxyethyl) | trans (5R, 6R) | 0 | 29 |

Als Testkeim wurde ein besonders starker Betalactamase-Bildner verwendet. E. coli W 31103 R6K (TEM I) wurde deshalb mehrfach in einer Kulturlösung mit 20 mcg/ml Cefaclor bebrütet. Cefaclor allein (30 mcg) ergab einen Hemmhof von 16 mm, mit 10 mcg Clavulansäure kombiniert einen solchen von 22 mm Durchmesser.

Mit einer Verbreitung von 75 % sind TEM I Enzyme die wichtigsten Betalactamasen. Sie treten bei einer Vielzahl von Enterobacteriaceae auf. Es zeigte sich, dass die Oxapenemcarbonsäuren Ib und Ie eine besonders gute Wirkung als Betalactamasehemmer gegen E. coli TEM I zeigen und das Antibiotikum Cefaclor wirksam gegen das bakterielle Enzym zu schützen vermögen. Ia und Id zeigen zwar eine gute Eigenwirkung als Antibiotika. Der Hemmhofzuwachs, d.h. die Schutzwirkung ist hier aber vergleichsweise gering.

Clavulansäure ist als potenter Penicillinase-Hemmer bekannt. Es wurde jedoch berichtet (Amer. J. Med. 79 (suppl. 5B), 194. (1985), dass sie nicht gegen Cephalosporinasen aus Enterobacter cloacae oder aus Pseudomonas aeruginosa wirkt. Verbindung Ie (5R,6R)-2-tert-Butyl-((1'S)-hydroxyethyl)oxapenem-3-carbonsäure (K-Salz) dagegen ist auch gegen diese Keime als Betalactamasehemmer wirksam. Entscheidend ist nur, dass das jeweilige Antibiotikum, das mit Ie kombiniert wird, in das jeweilige Bakterium einzudringen vermag.

Die erfindungsgemässen Verbindungen sind in der Lage, schon in geringen Konzentrationen die Hemmwirkung aller untersuchter Penicilline wie z. B. Amoxycillin, Piperacillin, Mezlocillin oder Cephalosporine wie z. B. Cefaclor, Cefoperazon gegen Betalactamase-bildende Bakterien zu verstärken. Auch gemeinhin als Betalactamase-stabile geltende Cephalosporine der dritten Generation wie z. B. Cefotaxim oder Ceftriaxon (je 30 mcg) ergaben in der Kombination mit Ie (10 mcg) erstmals deutliche Hemmhöfe vom Durchmesser 27 bzw. 26 mm gegen E. cloacae. Die erfindungsgemässen Verbindungen sind stark wirksam gegen Penicillinasen und Cephalosporinasen.

Die als Betalactamasehemmer hervorragenden Verbindungen Ib und Ie beinhalten eine Seitenkette, deren Konfiguration der jenigen von Thienamycin entgegengesetzt ist. Solche Oxapenemcarbonsäuren mit der Epithienamycin-Seitenkette sind daher für die Verwendung als Betalactamasehemmer bevorzugt. Besonders bevorzugt sind Verbindungen mit (5R,6R)-Konfiguration und ((1'S)-Hydroxyalkyl)-Gruppe als 6-Seitenkette.

Hydroxyalkyloxapenemcarbonsäuren der (5R)-Konfiguration sind an sich bekannt (EP 89117433.6). Sie wurden jedoch nur als Diastereoisomere isoliert und geprüft. Eine genauere Aussage über den Einfluss der Konfiguration der Hydroxyalkylseitenkette auf die Betalactamase-Hemmwirkung war auf dieser Basis nicht möglich. Isomerenreine (5R,6R)-Oxapenemcarbonsäuren sind zwar kürzlich beschrieben worden (M. Murakami, T. Aoki, M. Matsuura, W. Nagata, J. Antibiot. 1990, 1441 - 9 (Engl.). Sie enthielten aber ausschliesslich Hydroxyethyl-Seitenketten der für die Betalactamase-Hemmwirkung ungünstigen (1R)-Konfiguration.

Die als Betalactamasehemmer hervorragend wirksamen Verbindungen Ib und Ie sind nach dem Stand der Technik unbekannt. Ihre Wirkung als Betalactamasehemmer gegenüber Penicillinasen und Cephalosporinasen von Gram-positiven und Gram-negativen Bakterien ist signifikant und überraschend. Sie können als enantiomerenreine (6R,1'S)-Verbindungen oder in racemischer Form als Betalactamasehemmer verwendet werden.

Die erfindungsgemässen ((1'S)-Hydroxyalkyl)oxapenem-3-carbonsäuren bzw. ihre Salze können vorzugsweise mit oral applizierbaren Antibiotika wie z. B. Ampicillin, Amoxycillin, Cefaclor, Cefprozil, Cefuroxim-axetil, Cefixime, Cefpodoxime-proxetil, Cefteram-pivoxil, Cefetamet-pivoxil, Ceftibuten, Flomoxef o. ä. verabreicht werden. Aber auch Kombinationen mit parenteral applizierbaren Antibiotika wie z. B. Penicillin G, Piperacillin, Azlocillin, Mezlocillin, Ticarcillin, Mecillinam, Cefopearzon, Cephalothin, Cefazolin, Cephaloridin, Cefsudolin, Cefotaxim, Cefotiam, Ceftriaxon, Cefpirome, Cefepime o. ä. sind bevorzugt, aber auch Kombinationen mit nichtklassischen Betalactamantibiotika sind möglich.

Diese Kombination sind nützlich bei der Chemotherapie gegen alle bakteriellen Infektionskrankheiten, die üblicherweise mit Betalactamantibiotika bekämpft werden, besonders bevorzugt werden sie jedoch bei Kranheiten verwendet, die durch Penicillin-resistente und Cephalosporinresistente Keime verursacht werden. Beispiele solcher Bakterien sind Staph. aureus, H. influenzae, N. gonorrhoaeae, B. catarrhalis, Bacteroides fragilis, N. meningitidis, Citrobacter freundii, Enterobacter cloacae, Proteus mirabilis, Proteus vulgaris, Providentia rettgeri, Providentia stuarti, Pseudomonas aeruginosa, Salmonella, Serratia marescens, E. coli oder ähnlich.

Die jeweilige Dosis der erfindungsgemässen Betalactamasehemmer richtet sich im allgemeinen nach dem jeweiligen Antibiotikum und der zu behandelnden Infektionskrankheit. Diese Dosis kann unter Umständen gleich hoch oder ähnlich hoch sein wie die des jeweiligen Antibiotikums. Sie kann auch niedriger sein, besonders bei parenteraler Applikation. Das Verhältnis beträgt vorzugsweise 1 : 10 bis 1 : 1, aber auch andere Dosierunden sind möglich.

Die erfindungsgemässen ((1'S)-Hydroxyalkyl)oxapenem-3-carbonsäuren werden wie folgt hergestellt:

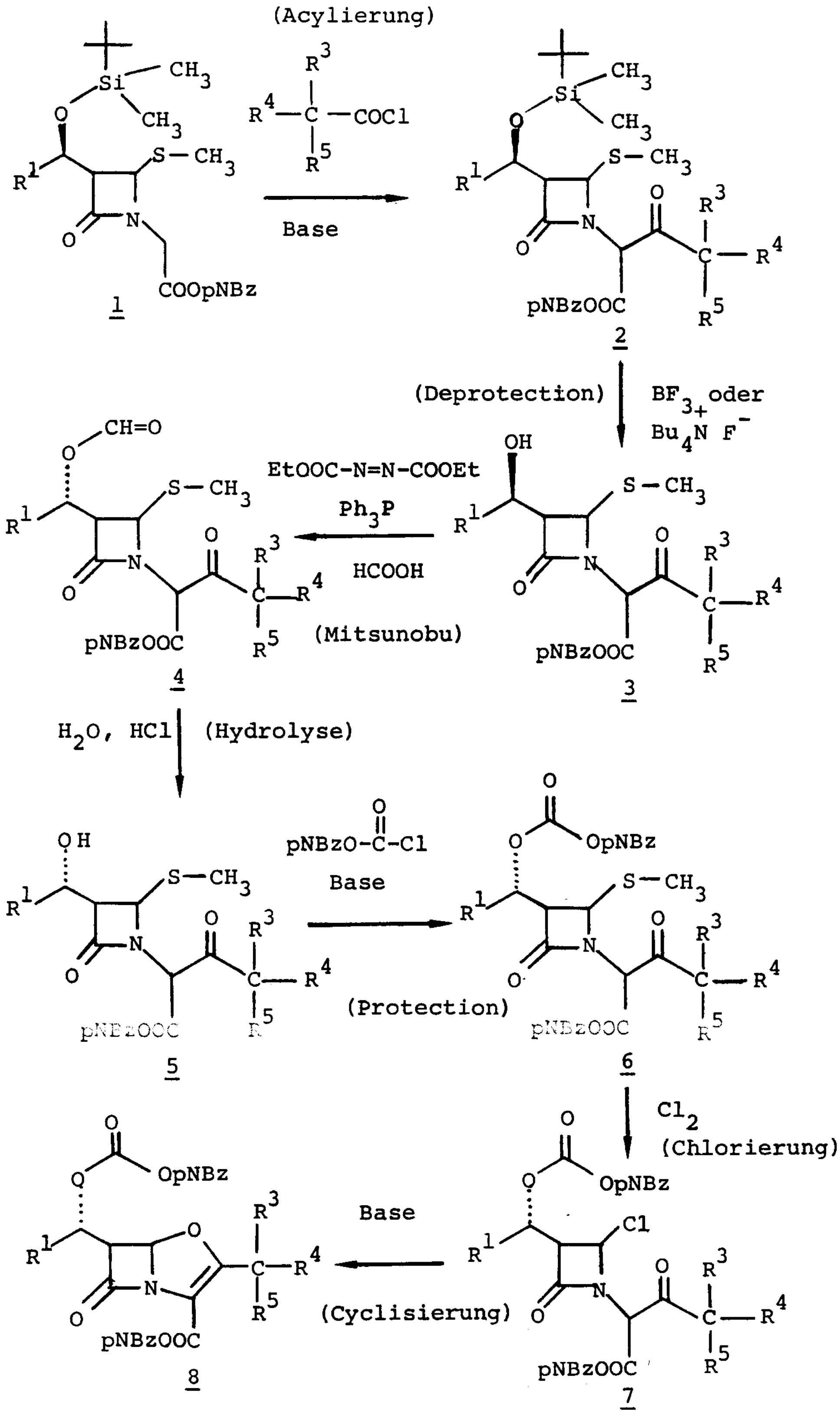

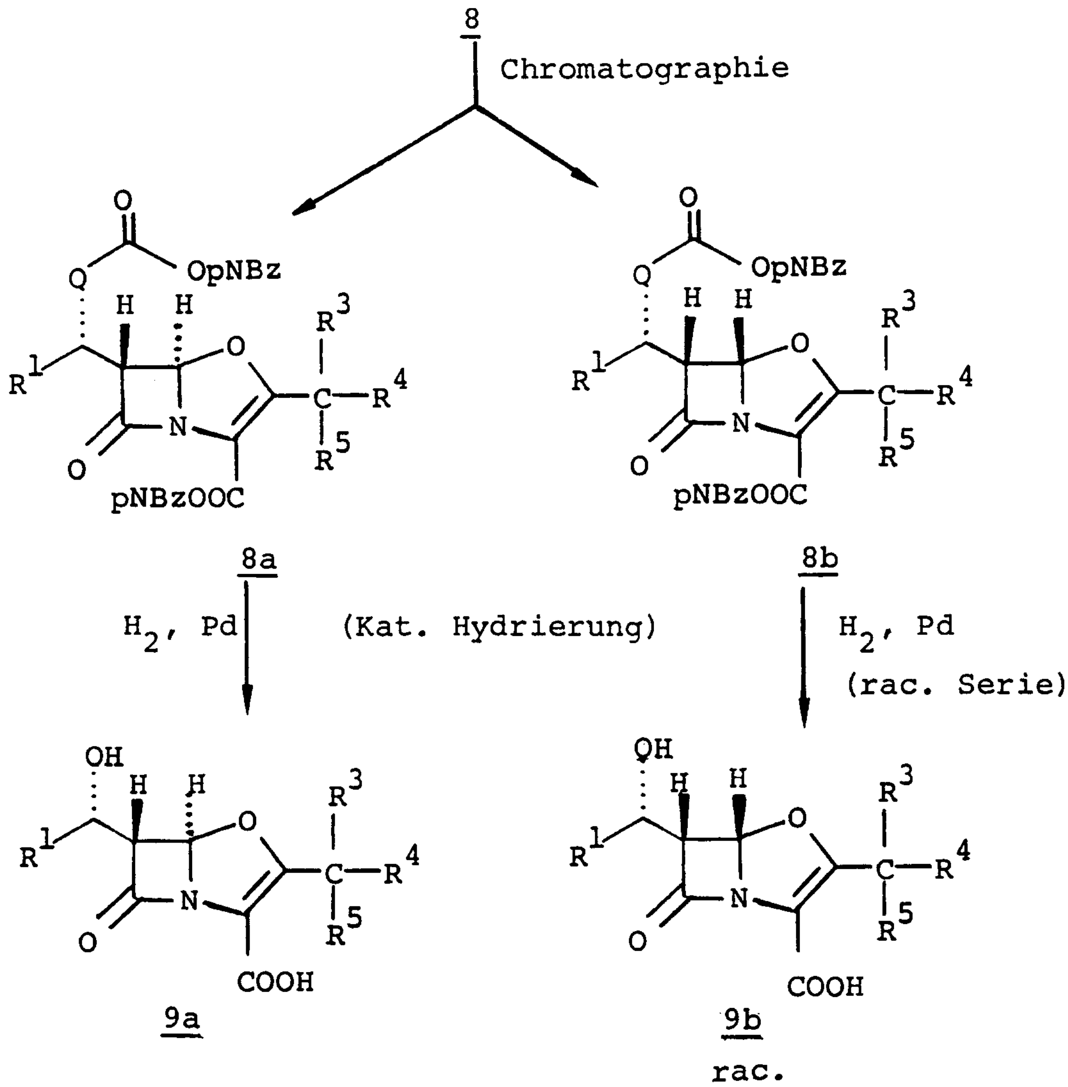


8

8a

8b

9a

9b

rac.

Ausgangsmaterialien vom Typ 1 sind beschrieben worden (J. Antibiot. 1990, 1441 - 9). Die Konfidurationsumkehr der Hydroxyalkylseitenkette von 3 erfolgt durch die Mitsunobu-Reaktion. Dieses Verfahren ist auf dem Gebiet der Carbapeneme erprobt worden (G.I. Georg, J. Kant, H.S. Gill, J. Amer. Chem Soc. 1987, 109, 1129 - 35). Das Gemisch der cis, trans-Isomeren 8 ist durch Chromatographie auf Kieselgel trennbar. In bestimmten Fällen ist eine Trennung auch durch fraktionierte Kristallisation möglich.

Ein alternatives Verfahren benützt (3R,4R)-4-Acetoxy-3-((1S-tert-butyldimethylsilyloxy)ethyl)azetidin-2-on als Ausgangsmaterial. Nach diesem Verfahren erübrigt sich die Konfigurationsumkehr in der Seitenkette; die Herstellung des Ausgangsmaterials 10 findet sich in J. Amer. Chem. Soc. 1987, 109, 1129 - 35. Die Herstellung erfolgt nach folgendem

Reaktionsschema:

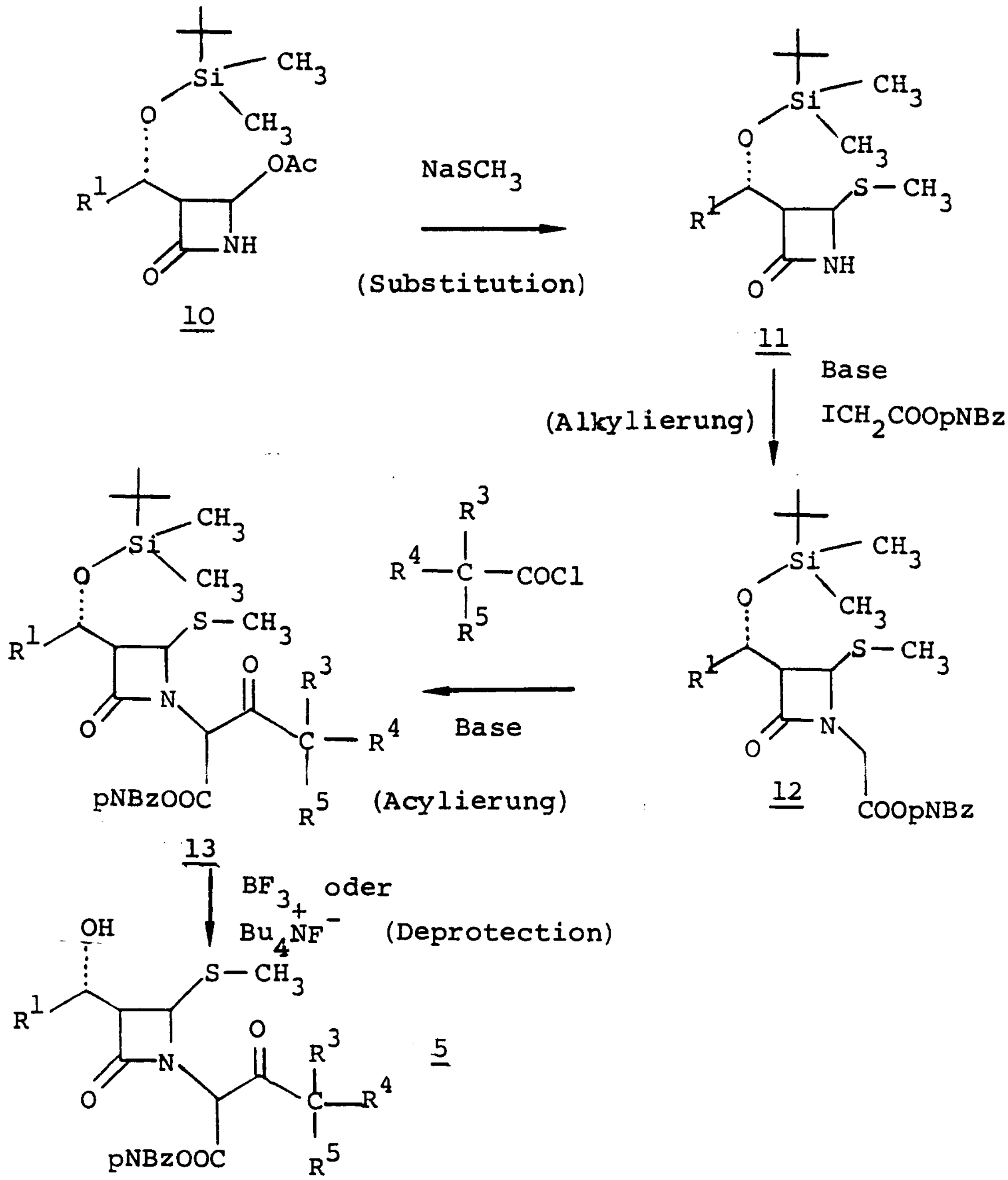


10 11 12 13 5

Die weiteren Umsetzungen von 5 zu 9a wurden im vorhergehenden Reaktionsschema beschrieben. Auf dem gleichen Reaktionsweg werden aus dem aus (S)-3-Hydroxybuttersäure gewonnenen (3S,4S)-4-Acetoxy-3-((1S)-tert-butyldimethylsilyloxy)ethylazetidin-2-on (J. Amer. Chem Soc. 1987, 109, 1132) (5R,6S)-(1'S-Hydroxyalkyl)oxapenem-3-carbonsäuren der like-cis Konfiguration erhalten.

Racemische Hydroxyalkyloxapenemcarbonsäuren mit der Konfiguration des Epithienamycins können ausgehend von threo- oder erythro-trans-3-(1-p-Nitrobenzyloxycarbonyloxyethyl)-4-(2-hydroxyethylsulfonyl)azetidin-2-on (J. Amer.

Chem. Soc. 1980, 102, 2039) als Ausgangsmaterial erhalten werden.

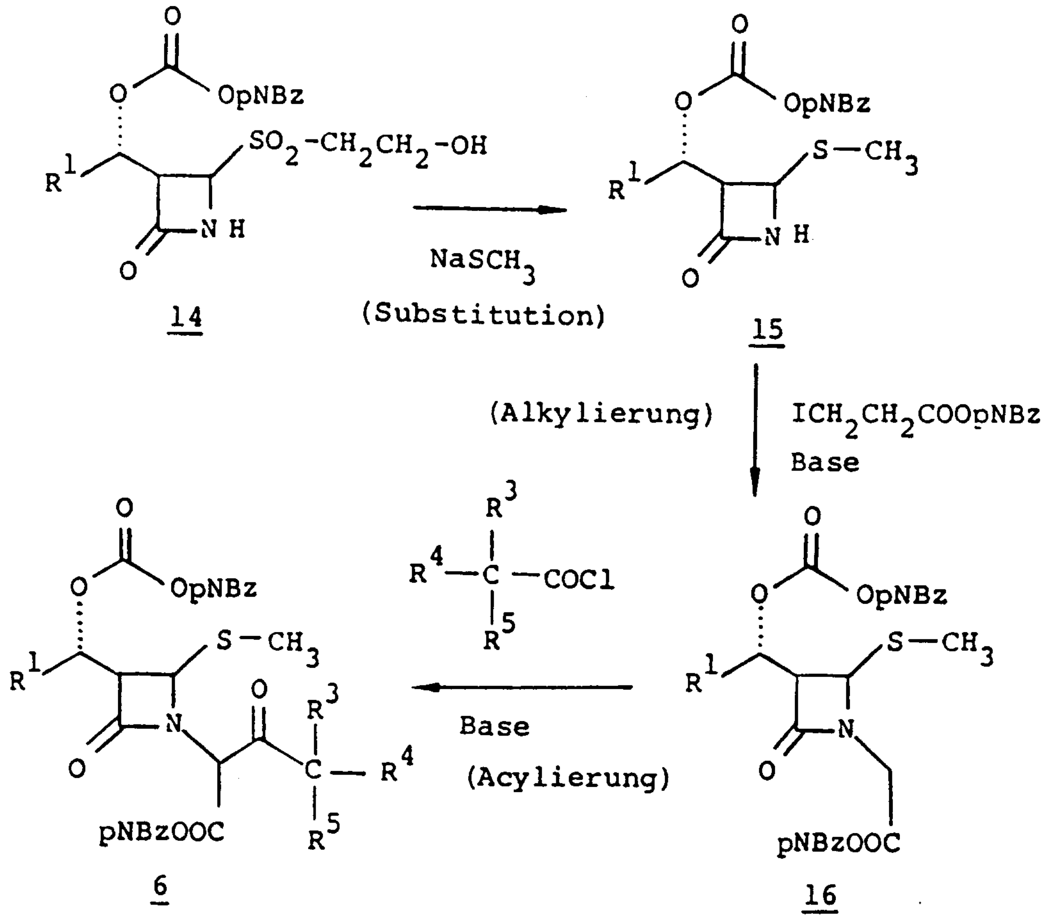


14 15 16 6

Die weiteren Umsetzungen von threo- oder erythro-6 erfolgen nach dem vorhergehenden Reaktionsschema, wobei aus threo-6 like-cis 9 und aus erythro-6 unlike-trans 9 entsteht.

Die in den Reaktionschemata gezeichnete p-Nitrobenzylschutzgruppe hat sich bei der Herstellung vieler stabiler Oxapenem-3-carbonsäuren bewährt. Diese Schutzgruppe lässt sich besonders leicht und unter milden Reaktionsbedingungen mit Hilfe von Wasserstoff und eines Hydrierkatalysators wie z. B. Palladium auf Aktivkohle in an sich bekannter Weise abspalten. Diese katalytische Hydrierung kann einphasig in einem Lösungsmittel wie z. B. Essigsäureethylester oder aber zweiphasig in einem Gemisch von Essigsäureethylester und Wasser in Gegenwart eines Alkali-oder Erdalkalihydrogencarbonats durchgeführt werden. Auf diese Weise wird die freie Carbonsäuregruppe der erfindungsmässigen Verbindungen direkt in das entsprechende Salz überführt.

Besonders bevorzugt ist die gemeinsame Verwendung einer p-Nitrobenzyl-Schutzgruppe für die Carboxylgruppe (p-Nitrobenzylester) und einer p-Nitrobenzylcarbonat-Schutzgruppe für die alkoholische Funktion der erfindungsgemässen Verbindungen. Dadurch können beide Schutzgruppen in einem Arbeitsgang, durch katalytische Hydrierung abgespalten werden.

An Stelle der im Reaktionsschema gezeichneten p-Nitrobenzyl-Schutzgruppe können auch andere mild abspaltbare Schutzgruppen verwendet werden. Dies wird dann notwendig, wenn die Gruppen $R^1$, $R^3$, $R^4$ oder $R^5$ Kohlenstoff-Kohlenstoff Doppelbindungen oder Dreifachbindungen enthalten. In diesem Falle ist die gleichzeitige Verwendung einer Allyl-Schutzgruppe für den Carboxylrest (Allylester) und einer Allylcarbonat-Schutzgruppe für die alkoholische Funktion bevorzugt. Die beiden Schutzgruppen werden dann im letzten Reaktionsschritt gemeinsam mit Tetrakis-triphenylphosphin-palladium und Triphenylphosphin in an sich bekannter Weise (M. Imuta, S. Uyeo, M. Nakano, T. Yoshida, Chem. Pharm. Bull. 33, 4371 -81 (1985)) abgespalten.

In der allgemeinen Beschreibung der vorliegenden Erfindung werden die Gruppen bevorzugt ausgewählt aus den pharmazeutisch annehmbaren, über C-C-Einfachbindungen an den übrigen Molekülteil gebundenen Gruppen, die enthalten: substituiertes oder unsubstituiertes Alkyl, Alkenyl, Alkinyl, Cycloalkyl, Alkylcycloalkyl, Alkylcycloalkenyl, Cycloalkylalkyl, Alkenylcycloalkyl, Cycloalkenylalkyl, Aryl, Aralkyl, Aralkenyl, Aralkinyl, Carboxy, Cyano, worin die vorhergehenden Alkyl, Alkenyl oder Alkinyl-Moleküle 1 bis 6 Kohlenstoffatome, die Cycloalkyl- oder die Cycloalkenyl-Molekülteile 3 bis 6 und die Aryl-Molekülteile 6 bis 10 Kohlenstoffatome enthalten, aromatisches oder aliphatisches Heterocyclyl, Heterocyclylalkyl, Heterocyclylalkenyl, Heterocyclylalkinyl, Alkylheterocyclyl, worin die vorhergehenden Alkyl-, Alkenyl- oder die Alkinyl-Molekülteile 1 bis 6 Kohlenstoffatome enthalten und der heterocyclische Molekülteil mono- oder bicyclisch ist und 3 bis 10 Ringatome enthält, wovon eines oder mehrere ausgewählt werden aus der Gruppe: Sauerstoff, Schwefel und Stickstoff, und wobei die Substituenten der oben aufgeführten Gruppen sein können: Geschütztes oder ungeschütztes Hydroxy, Hydroxyalkyl, Aminoalkyloxy, Alkyloxy, Acyloxy, Arylox, Heterocyclyloxy, Carbamoyl, Carbamoyloxy, Thiocarbamoyl, Thiocarbamoyloxy, Alkylcarbamoyloxy, Alkylthiocarbamoyloxy, Mercapto, Alkylthio, Hydroxyalkylthio, Aminoalkylthio, Amidinoalkylthio, Acylthio, Arylthio, Alkylheteroarylthio, Hydroxyalkylheteroarylthio, Heterocyclylthio, Carbamoylthio, Alkylcarbamoylthio, Thiocarbamoylthio, Alkylthiocarbamoylthio, geschütztes oder ungeschütztes Amino oder Monoalkylamino, Dialkylamino, Oxo, geschütztes oder ungeschütztes Oximino oder Alkylimino, Tetraalkylammonium, Cycloalkylamino, Arylamino, Heteroarylamino, Heterocyclylamino, Acylamino, Amidino, Alkylamidino, Guanidino, Alkylguanidino, Carbamoylamino, Alkylcarbamoylamino, Thiocarbamoylamino, Alkylthiocarbamoylamino, Nitro, Chlor, Brom, Fluor, Iod, Azido, Cyano, Alkylsulfinyl, Alkylsulfonyl, Sulfonamido, Sulfamoyloxy, Alkylsulfonyloxy oder geschütztes oder ungeschütztes Sulfo, Sulfoxy oder Carboxy, wobei die Substituenten unabhängig voneinander ein- oder mehrfach auftreten und ihr Alkyl-Molekülteil 1 bis 6 Kohlenstoffatome, ihr Aryl-Molekülteil 6 bis 10 Kohlenstoffatome enthält, und wobei der heteroaromatische oder heterocyclische Molekülteil mono- oder bicyclisch ist und 3 bis 10 Ringatome enthält, woven eines oder mehrere ausgewählt werden aus der Gruppe: Sauerstoff, Schwefel und Stickstoff, und $R^2$ bedeutet Wasserstoff, Alkyl-oder Acyl bis zu 10 Kohlenstoffatomen, Sulfo, Aminosulfonyl oder Phosphoroyl

Eine besonders bevorzugte Verbindungsklasse ist die, worin $R^1$ ausgewählt wird aus den Gruppen:
$CH_3$ $CH_2$-$CH_3$ $H_2C$=CH $H_2C$=CH-$CH_2$ HC≡C HC≡C-$CH_2$

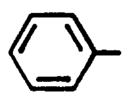
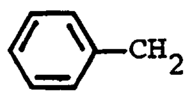
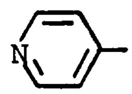
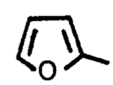

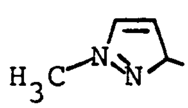
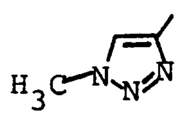
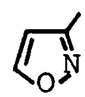
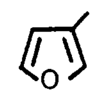

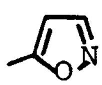
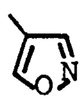
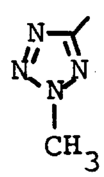
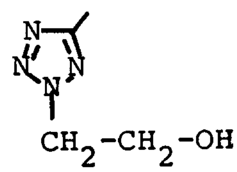

und $R^2$ Wasserstoff, Alkyl- oder Acyl mit bis zu 10 Kohlenstoffatomen, Sulfo, Aminosulfonyl oder Phosphoroyl bedeutet, $R^3$ und $R^4$ Methyl bedeuten und $R^5$ ausgewählt wird aus den Gruppen:

$CH_3$ $CH_2$-$CH_3$ $H_2C$=CH $H_2C$=CH-$CH_2$ HC≡C HC≡C-$CH_2$

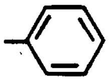 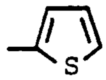 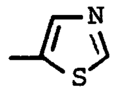

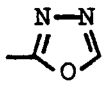 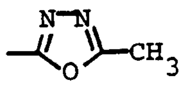 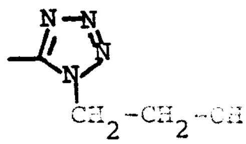

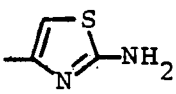 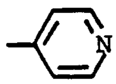 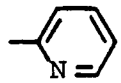

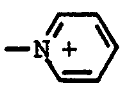 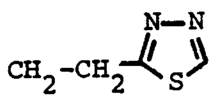 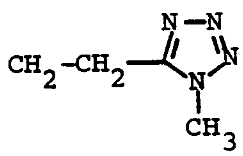

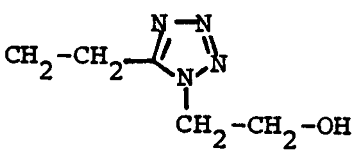 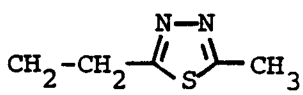

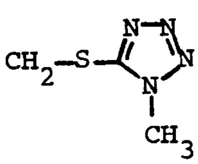 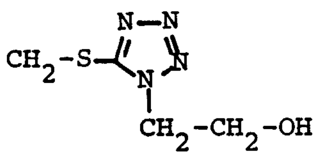 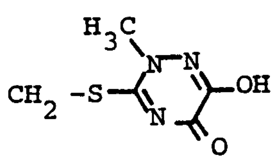

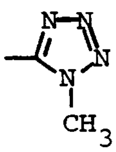 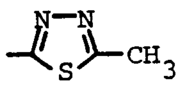 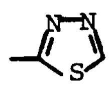

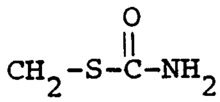

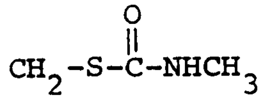

$CH_2$-S-$CH_3$ $CH_2$-S-$CH_2$-$CH_2$-$NH_2$

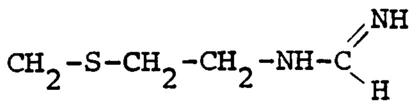

$CH_2$-$NH_2$

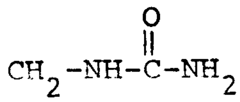

$CH_2$-NH-CHO

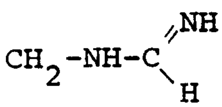

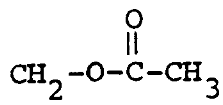

$CH_2$-O-CHO $CH_2$-Cl

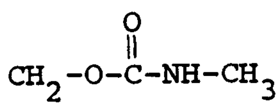

$CH_2$-$N_3$

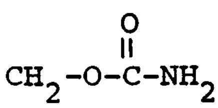

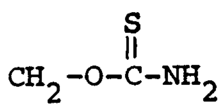

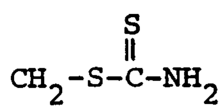

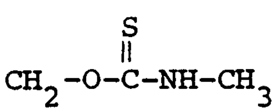

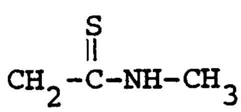

$CH_2$-$CH_2$-$NH_2$

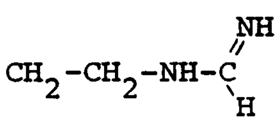

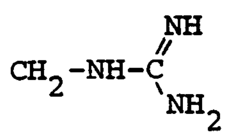

$CH_2-CH_2-CH_2-NH_2$

$$CH_2-CH_2-CH_2-NH-C(=NH)-H$$

$CH_2-CH_2-COOH$ $CH_2-COOH$ $CH_2-O-CH_3$

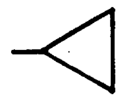

COOH $CH_2-OH$ $CH_2-CH_2-OH$

$$CH_2-CH_2-O-C(=O)-NH_2$$

C≡N

$$CH_2-CH_2-O-C(=O)-NHCH_3 \qquad CH_2-CH_2-CH_2-O-C(=O)-NH_2$$

$$CH_2-CH_2-CH_2-O-C(=O)-NH-CH_3$$

$CH_2-CH_2-NH-CHO$

Bevorzugte Schutzgruppen der geschützten Substituenten von $R^1$, $R^3$, $R^4$ und $R^5$ sind Benzyl, p-Nitrobenzyl, Benzyloxycarbonyl, p-Nitrobenzyloxycarbonyl, Allyl, Allyloxycarbonyl, Trimethylsilyl, tert-Butyldimethylsilyl, Benzyliden und Oxomethylen.

Die erfindungsgemässen Produkte bilden eine grosse Vielzahl pharmakologisch annehmbarer Salze mit anorganischen und organischen Basen. Diese umfassen beispielsweise Metallsalze, die sich von Alkalimetall- oder Erdalkalimetallhydroxiden, -carbonaten oder -bicarbonaten ableiten, und Salze, die sich von primären, sekundären oder tertiären Aminen ableiten, wie Monoalkylamine, Dialkylamine, Trialkylamine, niedrig-Alkanolamine, Di-niedrig-alkanolamine, niedrig-Alkylendiamine, N,N-Diaralkyl-niedrig-alkylendiamine, Aralkylamine, Amino-subst.-niedrig-alkanole, N,N-Di-niedrig-alkylamino-subst.-niedrig-alkanole, Amino-, Polyamino- und Guanidino-subst.-niedrig- Alkansäuren und Stickstoff enthaltende heterocyclische Amine. Beispiele für Salze sind solche, die sich von Natriumhydroxid, Natriumcarbonat, Natriumbicarbonat, Kaliumcarbonat, Kaliumhydroxid, Calciumcarbonat, Trimethylamin, Triäthylamin, Piperidin, Morpholin, Chinin, Lysin, Protamin, Arginin, Procain, Äthanolamin, Morphin, Benzylamin, Äthylendiamin, N,N'-Dibenzyläthylendiamin, Diäthanolamin, Piperazin, Dimethyl-aminoäthanol, 2-Amino-2-methyl-1-propanol, Theophyllin, N-Methylglucamin u.ä.

Gegenstand der Erfindung sind weiterhin Salze von Aminogruppen, die in bestimmten erfindungsgemässen Verbindungen in den Gruppen $R^1$, $R^3$, $R^4$ oder $R^5$ vorhanden sind. Solche pharmazeutisch annehmbaren Additionssalze leiten sich von organischen und anorganischen Säuren, wie HCl, HBr, Citronensäure, Weinsäure u.ä., ab.

Die Salze können Monosalze, wie das Mononatriumsalz, das durch Behandlung von 1 Äquiv. Natriumhydroxid mit 1 Äquiv. der erfindungsgemässen Verbindungen entsteht wie auch Disalze sein. Solche Salze können durch Behandlung mit 1 Äquiv. einer Base mit einem zweiwertigen Kation, wie Calciumhydroxid, mit 1 Äquiv. der erfindungsgemässen Stoffe erhalten werden. Die erfindungsgemässen Salze sind pharmakologisch annehmbare, nichttoxische Derivate, die als aktiver Bestandteil in geeigneten pharmazeutischen Dosiseinheitsformen verwendet werden können. Sie können ebenfalls mit anderen Arzneimitteln unter Bildung von Zubereitungen mit einem breiten Aktivitätsspektrum kombiniert werden.

Die neuen erfindungsgemässen Verbindungen sind wertvolle Betalactamasehemmer, die gegenüber verschiedenen grampositiven und gramnegativen wirksam sind. Die freie Säure und insbesondere ihre Salze, wie die Amin- und Metallsalze, insbesondere die Alkalimetall- oder die Erdalkalimetallsalze können zur Entfernung empfindlicher Pathogene von zahnmedizinischen und medizinischen Vorrichtungen, für die Abtötung von Mikroorganismen und für die therapeutische Verwendung bei Menschen und Tieren eingesetzt werden. Zu diesem letzteren Zweck werden pharmakologisch annehmbare Salze mit anorganischen und organischen Basen, wie sie an sich bekannt sind und bei der Verabreichung von Penicillinen und Cephalosporinen verwendet werden, verwendet. Beispielsweise können Salze, wie Alkalimetall- und Erdalkalimetallsalze, und primäre, sekundäre und tertiäre Aminsalze für diesen Zweck verwendet werden. Diese Salze können mit pharmazeutisch annehmbaren flüssigen und festen Trägern unter Bildung geeigneter Dosiseinheitsformen, wie Pillen, Tabletten, Kapseln, Suppositorien, Sirupen, Elixieren u.ä., verwendet werden, die nach an sich bekannten Verfahren hergestellt werden können.

Die neuen Verbindungen sind wertvolle Betalactamasehemmer gegen verschiedene grampositive und gramnegative Bakterien und finden dementsprechend in der Human- und Veterinärmedizin Verwendung. Die erfindungsgemässen Verbindungen können als antibakterielle Arzneimittel zur Behandlung von Infektionen verwendet werden, die durch grampositive oder gramnegative Bakterien erzeugt werden, z.B. gegen Staphylococcus aureus, Escherichia coli, Klebsiella pneumoniae, Bacillus subtilis, Salmonella typhosa, Pseudomonas und Bacterium proteus.

Die erfindungsgemässen Betalactamasehemmer können weiterhin als Zusatzstoffe für Tierfutter, für die Konservierung von Nahrungsmitteln bzw. Futter und als Desinfektionsmittel verwendet werden. Beispielsweise können sie in wässrigen Zubereitungen in Konzentrationen im Bereich von 0.1 bis 100 Teilen Antibiotikum/Million Teile Lösung zur Zerstörung und Inhibierung des Wachstums schädlicher Bakterien auf medizinischen und zahnmedizinischen Geräten und als Bakterizide bei industriellen Anwendungen, z.B. bei Anstrichmitteln auf Wassergrundlage und in weissem Wasser der Papiermühlen, zur Inhibierung des Wachstums schädlicher Bakterien verwendet werden.

Die erfindungsgemässen Produkte können allein oder zusammen als aktiver Bestandteil in irgendeiner Vielzahl von pharmazeutischen Zubereitungen verwendet werden. Diese Produkte und ihre entsprechenden Salze können in Kapselform oder als Tabletten, Pulver oder flüssige Lösungen oder als Suspensionen oder Elixiere verwendet werden. Sie können oral, intravenös oder intramuskulär verabreicht werden.

Bevorzugt sind dabei pharmazeutische Zubereitungen, die neben den erfindungsgemässen Betalactamasehemmern und an sich bekannten Zusatzstoffen auch ein Betalactamantibiotikum enthalten. Das Mischungsverhältnis zwischen Hemmer und Antibiotikum beträgt Vorzugsweise 1 : 10 bis 1 : 2, aber auch andere Dosierungen sind möglich.

Besonders bevorzugt sind dabei pharmazeutische Zubereitungen, die neben den erfindungsgemässen Betalactamasehemmern ein Penicillin oder ein Cephalosporin enthalten.

Die Zubereitungen werden bevorzugt in einer für die Absorption durch den gastro-intestinalen Trakt geeigneten Form verabreicht. Tabletten und Kapseln für die orale Verabreichung können in Dosiseinheitsform vorliegen und sie können übliche Arzneimittelträgerstoffe, wie Bindemittel, z.B. Sirup, Akaziengummi, Gelatine, Sorbit, Tragant oder Polyvinylpyrrolidon; Füllstoffe, z.B. Lactose, Zucker, Maisstärke, Calciumphosphat, Sorbit oder Glycin; Schmiermittel, z.B. Magnesiumstearat, Talk, Polyäthylenglykol, Siliciumdioxid; Desintegrationsmittel, z.B. Kartoffelstärke, oder annehmbare Benetzungsmittel, wie Natriumlaurylsulfat, enthalten. Die Tabletten können nach an sich gut bekannten Verfahren beschichtet werden. Orale, flüssige Zubereitungen können in Form von wässrigen oder Öligen Suspensionen, Lösungen, Emulsionen, Sirupen, Elixieren usw. vorliegen, oder sie können als Trockenprodukt, z.B. für die Rekonstitution mit Wasser oder anderen geeigneten Trägern vor der Verwendung, vorliegen. Solche flüssigen Präparationen können an sich bekannte Zusatzstoffe, wie Suspensionsmittel, z.B. Sorbitsirup, Methylcellulose, Glucose/Zuckersirup, Gelatine, Hydroxyäthylcellulose, Carboxymethylcellulose, Aluminiumstearatgel, oder hydrierte geniessbare Öle, z.B. Mandelöl, fraktioniertes Cocosnussöl, ölige Ester, Propylenglykol oder Äthylalkohol; Konservierungsmittel, z.B. Methyl- oder Propyl-p-hydroxybenzoat oder Sorbinsäure, enthalten. Suppositorien werden an sich bekannte Suppositoriengrundstoffe, z.B. Kakaobutter oder andere Glyceride, enthalten.

Die Zubereitungen für die Injektion können in Dosiseinheitsform in Ampullen ode in Behältern mit mehreren Dosen mit einem zugegebenen Konservierungsstoff vorliegen. Die Zubereitungen können in Form von Suspensionen, Lösungen oder Emulsionen in öligen oder wässrigen Trägern vorliegen, und sie können Formulationsmittel, wie Suspensions-, Stabilisations- und/oder Dispersionsmittel enthalten. Alternativ kann der aktive Bestandteil in Pulverform für die Rekonstitution mit einem geeigneten Träger, z.B. sterilem, pyrogenfreiem Wasser, vor der Verwendung vorliegen.

Die Zubereitungen können ebenfalls in einer für die Absorption durch die Schleimhautmembranen der Nase und des Halses oder der Bronchialgewebe geeigneten Form vorliegen, und sie können zweckdienlich in Form von Pulvern oder flüssigen Sprays oder Inhalationsmitteln, Lutschbonbons, als Auspinselungsmittel für den Hals, etc. vorliegen. Für die Medikation der Augen und Ohren können die Zubereitungen in Form einzelner Kapseln in flüssiger oder semi-fester Form vorliegen, oder sie können als Tropfen usw. verwendet werden. Topische Anwendungen können in hydrophoben oder hydrophilen Grundstoffen als Salben, Cremes, Lotionen, Auspinselungsmittel, Pulver usw. vorliegen bzw. formuliert werden.

Die erfindungsgemässen Zubereitungen können zusätzlich zu dem Träger einen anderen Bestandteil, wie Stabilisatoren, Bindemittel, Antioxydantien, Konservierungsmittel, Schmiermittel, Suspensionsmittel, Viskositätsmittel oder

Geschmacksmittel u.ä., enthalten. Zusätzlich können in den Zubereitungen andere aktive Bestandteile enthalten sein, so daß man ein breiteres antibiotisches Aktivitätsspektrum erhält.

Für die Veterinärmedizin können die Zubereitungen z.B. als intramammare Zubereitung in entweder langwirkenden oder schnellfreisetzenden Grundstoffen formuliert werden.

Die zu verabreichende Dosis hängt in grossem Ausmass von dem Zustand des zu behandelnden Subjekts und dem Gewicht des Wirts, dem Weg und der Frequenz der Verabreichung ab. Der parenterale Weg ist für generalisierte Infektionen und der orale Weg für intestinale Infektionen bevorzugt. Im allgemeinen enthält eine tägliche orale Dosis etwa 1 bis etwa 200 mg aktiven Bestandteil/kg Körpergewicht des Subjekts bei einer oder mehreren Anwendungen pro Tag. Eine bevorzugte tägliche Dosis für erwachsene Menschen liegt im Bereich von etwa 5 bis 100 mg aktiven Bestandteil/kg Körpergewicht.

Die erfindungsgemässen Zubereitungen können in verschiedenen Einheitsdosisformen, z.B. in festen oder flüssigen, oral aufnehmbaren Dosisformen, verabreicht werden. Die Zubereitungen pro Einheitsdosis können entweder in fester oder in flüssiger Form 0.1 bis 99 % aktives Material enthalten. Der bevorzugte Bereich beträgt etwa 10 bis 60 %. Die Zubereitungen werden im allgemeinen 15 bis etwa 1500 mg aktiven Bestandteil enthalten, im allgemeinen ist es jedoch bevorzugt, eine Dosismenge im Bereich von etwa 50 bis 500 mg zu verwenden. Bei der parenteralen Verabreichung wird die Einheitsdosis normalerweise die reine Verbindung in einer sterilen Wasserlösung sein oder in Form eines löslichen Pulvers, das aufgelöst werden kann, vorliegen.

Die folgenden Beispiele erläutern die erfindungsgemässen Produkte, Verfahren, Zubereitungen und Behandlungsverfahren.

Beispiel 1

Herstellung von (5R,6R)-trans-2-tert-butyl-6-((1'S)-hydroxyethyl)oxapenem-3-carbonsäure und ihrem Kaliumsalz

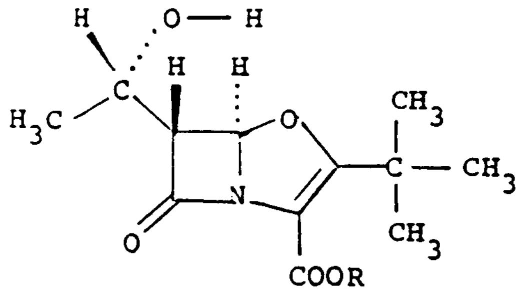

R = H, K

Acylierung: Zwischenprodukt 2 ($R^1$, $R^3$, $R^4$, $R^5$ = $CH_3$)

In einem Dreihalskolben mit mechanischem Rührer, Gummiseptum wird unter Stickstoff das Edukt 1 ($R^1$, $R^3$, $R^4$, $R^5$, = $CH_3$) (35.5 g) in 980 ml trockenem Tetrahydrofuran gelöst und die Lösung auf -78°C abgekühlt. Unter Rühren wird dann 9.8 ml Pivalinsäurechlorid zugespritzt und anschliessend 165 ml eine 1 molaren Lösung von Lithium-bis-trimethylsilyl amid zugegeben. Das Gemisch wird noch während 30 Minuten bei -78°C weitergerührt und dann zu 5 l Toluol gegossen. Die erhaltene Lösung wird dann nacheninander mit 3 l 1N HCl, 3 l gesättigte NaCl und nochmals mit 1 lt gesättigter NaCl-Lösung gewaschen und die organische Phase über Magnesiumsulfat getrocknet. Entfernen des Lösungsmittels im Vakuum und Chromatographie des Rückstands auf 1 kg Kieselgel mit Toluol-Ethylacetat 9 : 1 ergibt reines 2 als viskoses Öl (36.2g, 86 %). IR-Spektrum in $CH_2Cl_2$: 2920, 2850, 1760, 1750, 1705, 1605, 1525, 1350, 840 $cm^{-1}$.

Deprotection: Zwischenprodukt 3 ($R^1$, $R^3$, $R^4$, $R^5$ = $CH_3$)

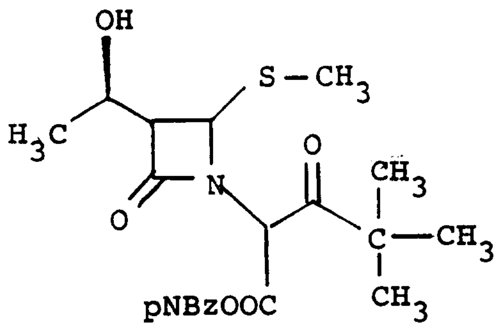

In einem Zweihals-Rundkolben mit Gummiseptum, Rückflusskühler und Magnetrührer wird zu einer Lösung von 2 ($R^1$, $R^3$, $R^4$, $R^5$ = $CH_3$) (5.53 g) in 48 ml trockenem Tetrahydrofuran 5.71 ml Essigsäure und 26.7 ml einer 1.1 molaren Lösung von Tetrabutylammoniumfluorid in THF zugespritzt. Das Septum wird dann durch einen Stopfen ersetzt und das Gemisch während 22 Stunden unter Stickstoff magnetisch gerührt. Anschliessend wird noch während 90 Minuten am Rückfluss gekocht. Das Reaktionsgemisch wird mit 1 lt Toluol verdünnt und nacheinander mit 400 ml gesättigter NaCl, 150 ml 2N $KHCO_3$-Lösung und nochmals mit 150 ml gesättigter NaCl gewaschen. Die organische Phase wird über Magnesiumsulfat getrocknet und das Lösungsmittel im Vakuum entfernt. Der kristalline Rückstand wird auf 140 g Kieselgel mit Toluol-Ethylacetat (3 : 1) chromatographiert, wobei 3.57 g 3 (82 %) kristallines Produkt erhalten werden. IR-Spektrum in $CH_2Cl_2$: 3600, 2960, 1765, 1750, 1710, 1610, 1525, 1350 $cm^{-1}$.

Mitsunobu-Reaktion: Zwischenprodukt 4 ($R^1$, $R^3$, $R^4$, $R^5$ = $CH_3$)

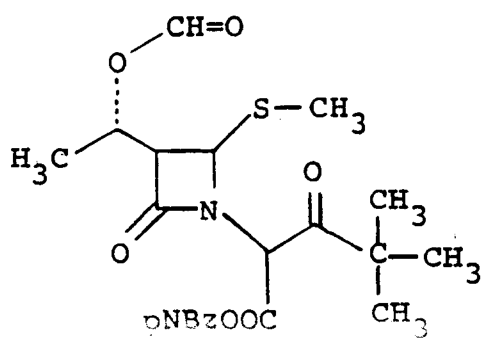

Zu einer Lösung von 3 (4.2 g) und Triphenylphosphin (5.03 g) in 72 ml trockenem Tetrahydrofuran wird unter Argon nacheinander 1.09 ml Ameisensäure und 3.78 ml Azodicarbonsäurediisopropylester bei 0°C gegeben und das Reaktionsgemisch während 45 Minuten bei 0°C und anschliessend noch 90 Minuten bei Raumtemperatur gerührt. Die Reaktionslösung wird mit 720 ml Toluol verdünnt und dann nacheinander mit je 720 ml Wasser, verdünnter $NaHCO_3$-Lösung und 10 %-iger NaCl-Lösung gewaschen. Die organische Phase wird über Magnesiumsulfat getrocknet und das Lösungsmittel im Vakuum entfernt. Chromatographie des Rohproduktes auf 200 g Kieselgel (0.040 - 0.063 mm) mit Toluol-Ethylacetat (97 : 3) und mit Toluol-Ethylacetat (95 : 5) ergibt 1.95 g (44 %) eines leicht gelblichen Öls. IR-Spektrum in $CH_2Cl_2$ : 2930, 1765, 1750, 1720, 1605, 1525, 1350, 1175 $cm^{-1}$.

Hydrolyse: Zwischenprodukt 5 ($R^1$, $R^3$, $R^4$, $R^5$ = $CH_3$)

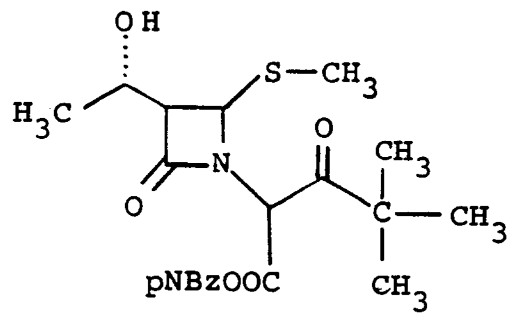

Zu einer Lösung von 4 ($R^1$, $R^3$, $R^4$, $R^5$ = $CH_3$) (1.81 g) in 39 ml Methanol wird bei 0°C unter Rühren 3.88 ml 1.0 N wässrige HCl getropft und das Gemisch während 9 Stunden bei Raumtemperatur gerührt. Das Reaktionsgemisch wird mit 200 ml Toluol verdünnt und nacheinander mit je 100 ml verdünnter $NaHCO_3$-Lösung und 10 %-iger NaCl-Lösung gewaschen. Die organische Phase wird über Magnesiumsulfat getrocknet und das Lösungsmittel im Vakuum entfernt. Nach Trocknen im Hochvakuum wird 1.70 g eines farblosen nichtkristallinen Feststoffes erhalten (100 %). Das Rohprodukt 4 wird ohne weitere Reinigung verwendet. IR-Spektrum in $CH_2Cl_2$: 3600, 2970, 1765, 1755, 1715, 1610, 1525, 1350 $cm^{-1}$.

Protection: Zwischenprodukt 6 ($R^1$, $R^3$, $R^4$, $R^5$ = $CH_3$)

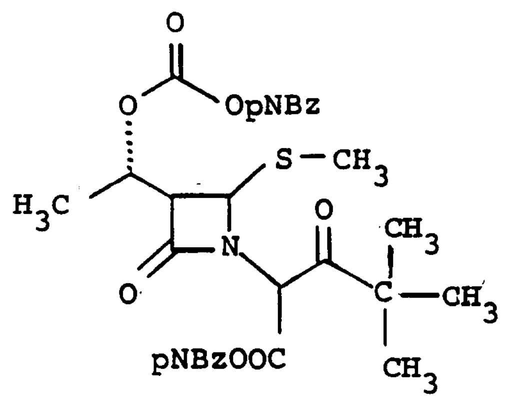

Zu einer Lösung von 5 ($R^1$, $R^3$, $R^4$, $R^5$ = $CH_3$) (1.63 g) in 8 ml Methylenchlorid wird bei -8 °C unter Rühren eine Lösung von Chlorameisensäure-p-nitrobenzylester (1.10 g) in 3 ml Methylenchlorid zugetropft und anschliessend innerhalb von 15 Minuten festes N,N-Dimethylaminopyridin (0.62 g) zugegeben. Nach 5 Minuten Rühren entsteht ein weisser Niederschlag. Das Reaktionsgemisch wird während 2 Stunden bei -5°C gerührt und anschliessend mit 75 ml Methylenchlorid verdünnt und mit 50 ml 10 %-iger NaCl-Lösung gewaschen. Die organische Phase wird über Magnesiumsulfat getrocknet und das Lösungsmittel im Vakuum entfernt. Das Rohprodukt von 6 wird dann auf 70 g Kieselgel mit Toluol-Ethylacetat (9 : 1) chromatographiert, wobei 1.87 g (81 %) reines 6 als nichtkristallinen Feststoff erhalten werden. IR-Spektrum in $CH_2Cl_2$: 2970, 1765, 1755, 1715, 1610, 1525, 1350, 1245, 850 $cm^{-1}$.

Chlorierung: Zwischenprodukt 7 ($R^1$, $R^3$, $R^4$, $R^5$ = $CH_3$)

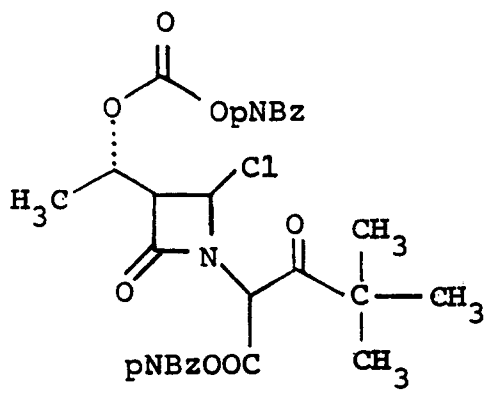

In einem 250 ml-Zweihalskolben mit Gummmiseptum, Stickstoff-Ballon und magnetischem Rührer wird bei -40°C unter Rühren zu einer Lösung von 6 ($R^1$, $R^3$, $R^4$, $R^5$ = $CH_3$) (1.77 g) in 115 ml Methylenchlorid mit einer Gasspritze 129 ml Chlorgas in die Reaktionslösung gespritzt und das Gemisch anschliessend während 10 Minuten gerührt. Die kalte Reaktionsmischung wird dann auf eine wässrige Lösung von 5.74 g wasserfreiem Natriumhydrogensulfit und 4.57 g wasserfreiem Natriumcarbonat gegossen und das Gemisch geschüttelt. Die wässrige Phase wird mit wenig Methylenchlorid extrahiert und die Extraktionslösungen vereinigt. Trocknen über Magnesiumsulfat und Entfernen des Lösungsmittels im Vakuum ergibt ein farbloses Rohprodukt 7. Chromatographie auf 4.3 g Kieselgel bei 0°C mit Toluol (10 ml Fraktionen) und mit Toluol-Ethylacetat (10 ml Fraktionen) liefert 1.49 g reines Produkt 7 (85 %) als nichtkristallinen weissen Festkörper.
IR-Spektrum in $CH_2Cl_2$: 2970, 1790, 1755, 1720, 1610, 1525, 1350, 1245, 850 $cm^{-1}$.

Cyclisierung: (5R,6R) und (5S,6R)-2-tert-Butyl-6-((1'S)-p-nitrobenzyloxycarbonyloxyethyl)oxapenem-3-carbonsäure-p-nitrobenzylester 8 ($R^1$, $R^3$, $R^4$, $R^5$ = $CH_3$)

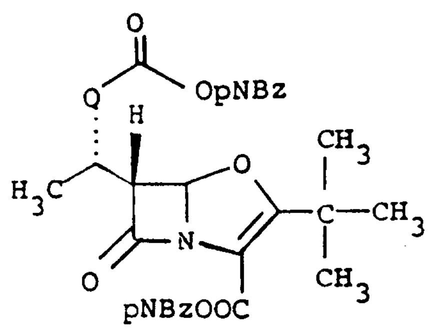

In einem Zweihalskolben mit Gummiseptum, Stickstoff-Ballon und magnetischem Rührer wird unter Rühren bei -30°C zu einer Lösung von 7 ($R^1$, $R^3$, $R^4$, $R^5$ = $CH_3$) (1.35 g) in 45 ml trockenem Tetrahydrofuran langsam mit einer Spritze 2.55 ml einer 0.92 molaren Lösung von Kalium-tert-butanolat in tert. Butanol zugegeben und das Reaktionsgemisch anschliessend während 90 Minuten bei -30°C gerührt. Die Reaktionslösung wird mit 240 ml Ethylacetat verdünnt und anschliessend nacheinander mit 220 ml 10 %-iger NaCl, 100 ml 10 %-iger NaCl und 100 ml gesättigter NaCl-Lösung gewaschen. Trocknen über Magnesiumsulfat und Entfernen des Lösungsmittels im Vakuum ergibt ein cis, trans-Isomerengemisch von 8. IR-Spektrum in $CH_2Cl_2$: 2970, 1800, 1750, 1715, 1605, 1580, 1525, 1350, 1315, 1240, 1090, 850 $cm^{-1}$.

Chromatographische Trennung von (5R,6R) und (5S,6R)-2-tert-Butyl-6-((1'S)-p-nitrobenzyloxycarbonyloxyethyl)oxapenem-3-carbonsäure-p-nitrobenzylester 8a und 8b ($R^1$, $R^3$, $R^4$, $R^5$ = $CH_3$)

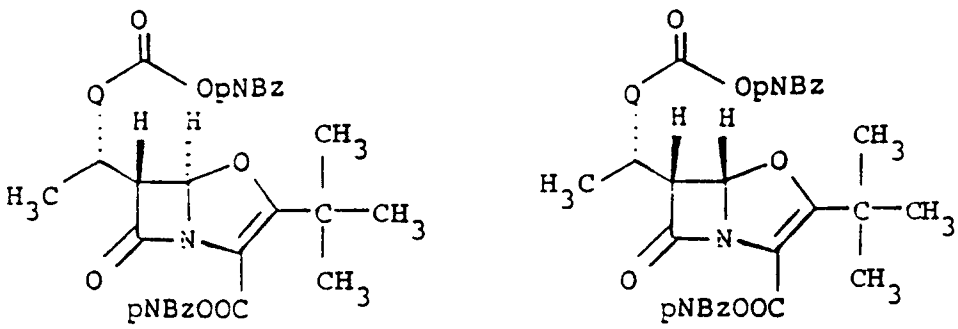

8a 8b

Das cis, trans-Isomerengemisch 8 ($R^1$, $R^3$, $R^4$, $R^5$ = $CH_3$) (1.25 g) wird bei -12°C auf 70 g Kieselgel (5 - 20 um) mit Hexan-Ethylacetat (1 : 1) chromatographiert, wobei 705 mg (56 %) reines 8a und 352 (28 %) 8b, das noch etwas 8a enthält, erhalten werden. Die jeweiligen Fraktionen werden bei 0°C im Hochvakuum bei 0.5 Torr eingedampft. 8a: $[\alpha]_D^{25}$ = +50° (c = 1, Ethylacetat).

8a: NMR in $CD_3CN$: δ = 1.3 (s, 9H), 1.43 (d, 3H, J = 6.5 Hz), 3.99 (dd, 1H, J = 1 Hz, J = 3.5 Hz), 5.19 (m, 1H), 5.26 (s, 2H), 5.33 (ABq, 2H, J = 14 Hz, J = 85 Hz), 5.78 (d, 1H, J = 1 Hz), 7.67 bis 7.58 (2d, 4H, J = 8.4 Hz), 8.19 (2d, 4H, J = 8.4 Hz).

8b: NMR in $CD_3CN$: δ = 1.23 (s, 9H), 1.42 (d, 3H, J = 6.5 Hz), 4.19 (m, 1H), 5.19 (m, 1H) 5.24 (s, 1H), 5.33 (ABq, 2H, J = 14 Hz, J = 82.6 Hz), 5.89 (d, 1H, J = 3.1 Hz), 7.59 bis 7.66 (2d, 4H, J = 9 Hz), 8.21 (2d, 4H, J = 9 Hz).

Trennung durch fraktionierte Kristallisation von (5R, 6R) und (5S,6R)-2-tert-Butyl-6-((1'S)-p-nitrobenzyloxycarbonyloxyethyl)oxapenem-3-carbonsäure-p-nitrobenzylester 8a und 8b ($R^1$, $R^3$, $R^4$, $R^5$ = $CH_3$)

Das cis-trans-Isomerengemisch 8 ($R^1$, $R^3$, $R^4$, $R^5$) (1.30 g) wird in 5 ml Ethylacetat gelöst und bis zur Trübung mit n-Hexan versetzt und zwei Tage bei -20°C stehen gelassen, wobei das Isomere 8b, das noch wenig 8a enthält, auskristallisiert. Das Kristallisat wird abgenutscht und die Mutterlauge sofort im Hochvakuum bei 0.5 Torr eingedampft, wobei reines 8a als weisser nichtkristalliner Festkörper (775 mg 61 %) zurückbleibt.

Isomerisierung von (5S,6R) zu (5R,6R)-2-tert-Butyl-6-((1'S)-p-nitrobenzyloxycarbonyloxyethyl)oxapenem-3-carbonsäure-p-nitrobenzlester 8b zu 8a ($R^1$, $R^3$, $R^4$, $R^5$ = $CH_3$)

8b ($R^1$, $R^3$ $R^4$, $R^5$ = $CH_3$) (510 mg) wird in Ethylacetat (10 ml) gelöst und während drei Tagen bei Raumtemperatur stehen gelassen, wobei sich das thermodynamische Gleichgewicht von 69 % 8a und 31 % 8b einstellt. Durch Chromatographie bei -15°C oder durch fraktionierte Kristallisation gemäss der vorangehenden Vorschriften wird weiteres reines Isomer 8a gewonnen (255 mg, 50 %).

Katalytische Hydrierung: (5R,6R)-2-tert-Butyl-((1'S)-6-hydroxyethyl)oxapenem-3-carbonsäure-Kaliumsalz 9a ($R^1$, $R^3$, $R^4$, $R^5$ = $CH_3$)

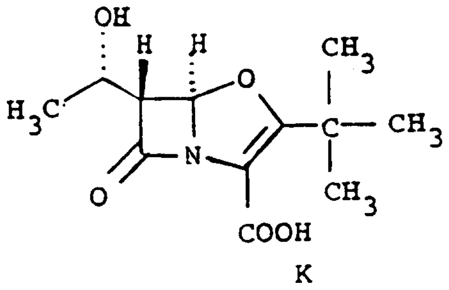

In einer Hydrierapparatur mit Gummiseptum wird 620 mg Pd auf Aktivkohle (10 %) in 48 ml Ethylacetat bei 0°C vorhydriert während 7 Minuten und anschliessend eine frisch zubereitete Lösung von 8a ($R^1$, $R^3$, $R^4$, $R^5$ = $CH_3$) (618 mg) in 6 ml Ethylacetat zugespritzt und während 30 Minuten bei 0°C hydriert. Innerhalb von 20 Minuten werden 210 ml Wasserstoff aufgenommen. Der Katalysator wird durch Abnutschen entfernt und das Filtrat bei 0°C sorgfältig mit einer gekühlten Lösung von 81 mg Kaliumhydrogencarbonat in doppelt destilliertem Wasser (20 ml) extrahiert. Die wässrige Phase wird noch zweimal mit Portionen von Ethylacetat (10 ml) gewaschen, kurz im Vakuum von Ethylacetat befreit und bei -25°C lyophilisiert bei 0.001 Torr, wobei 188 mg (59 %) Kaliumsalz 9a als weisses voluminöses Pulver erhalten werden.
UV-Spektrum in $H_2O$: $_{max}$ = 262 nm. NMR-Spektrum in $D_2O$: δ = 1.25 (s, 9H), 1.34 (d, 3H, J = 6.3 Hz), 3.84 (d, 1H, 4.6 Hz), 4.3 (m, 1H), 5.73 (s, 1H).

Beispiel 2

Herstellung von racemischer cis- und trans-2-tert-Butyl-6-((1'S)-hydroxyethyl)oxapenem-3-carbonsäure und ihrem Kaliumsalz.

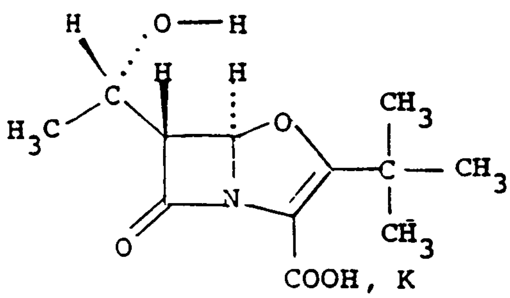

R = H, K

rac. trans

Substitution: Zwischenprodukt 15 ($R^1$= $CH_3$)

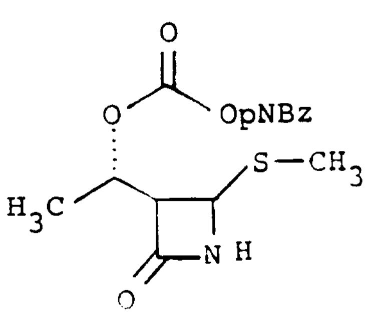

Unter heftigem Rühren wird zu einer Suspension von (erythro-trans)- 14 ($R^1 = CH_3$) (3.56 g) in Acetonitril (9.7 ml) eine Lösung von Natriummethanthiolat (0.93 g) in Wasser (9.7 ml) bei 0°C innerhalb von 15 Minuten zugetropft. und das Gemisch noch weitere 30 Minuten bei 0°C gerührt. Die gelbe, trübe Reaktionslösung wird dann mit 75 ml Methylenchlorid verdünnt und mit 25 ml Wasser gewaschen. Die wässrige Phase wird mit drei Portionen von je 20 ml Methylenchlorid extrahiert und die vereinigten organischen Phasen mit 30 ml gesättigter NaCl-Lösung gewaschen. Die organische Phase wird über Magnesiumsulfat getrocknet und das Lösungsmittel im Vakuum entfernt. Das erhaltene Rohprodukt 15 wird anschliessend auf 200 g Kieselgel mit Toluol-Ethylacetat (9 : 1) und Toluol Ethylacetat (4 : 1) chromatographiert, wobei 2.45 g (82 %) kristallines Produkt 15 (erythro, trans) erhalten werden. IR-Spektrum in $CH_2Cl_2$: 3400, 3060, 2990, 1775, 1755, 1610, 1525, 1350, 1245, 850 erhalten werden.

Alkylierung: Zwischenprodukt 16 ($R^1 = CH_3$)

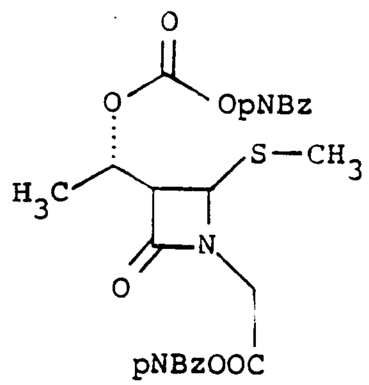

Zu einer Lösung von 15 (erythro-trans) ($R^1 = CH_3$) (2.30 g) in Acetonitril (23 ml) wird unter Rühren bei 0°C innerhalb von 5 Minuten Iodessigsäure-p-nitrobenzylester (3.26 g) gegeben und anschliessend innerhalb von 15 Minuten festes Cäsiumcarbonat (3.30 g) unter heftigem Rühren bei 0°C eingetragen und anschliessend das Reaktionsgemisch noch 1 Stunde gerührt. Das Gemisch wird mit 230 ml Toluol versetzt und nacheinander mit je 80 ml gesättigter Natriumhydrogencarbonat- und gesättigter Natriumchloridlösung gewaschen. Die organische Phase wird über Magnesiumsulfat getrocknet und das Lösungsmittel im Vakuum entfernt. Das erhaltene Rohprodukt wird dann auf 220 g Kieselgel mit Toluol-Ethylacetat (9 : 1) chromatographiert, wobei 2.21 g eines farblosen Festkörpers erhalten werden (62 %). IR-Spektrum in $CH_2Cl_2$: 3060, 1770, 1750, 1610, 1525, 1350, 1245, 1190 $cm^{-1}$.

Acylierung: Zwischenprodukt 6 ($R^1$, $R^3$, $R^4$, $R^5 = CH_3$)

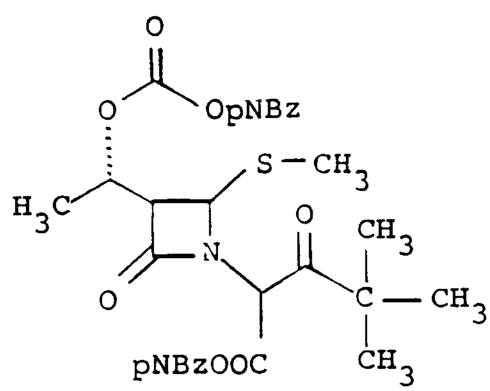

Zu einer Lösung von 16 (erythro), (2.21 g) und Pivaloylchlorid (0.54ml) in 53 ml Tetrahydrofuran wird bei -70°C unter Stickstoff und unter heftigem Rühren eine 1M Lösung (8.28 ml) von Lithium-bis-trimethylsilylamid in THF innerhalb von 15 Minuten zugetropft und das Reaktionsgemisch noch 30 Minuten bei -70°C gerührt. Die Reaktionslösung wird mit 350 ml Toluol verdünnt, mit 300 ml 2N HCl und zweimal mit je 300 ml gesättigter NaCl-Lösung gewaschen. Trocknen

der organische Phase über $MgSO_4$ und Entfernen des Lösungsmittels ergibt das Rohprodukt 6. Chromatographie auf Kieselgel (100 g) mit Toluol-Ethylacetat (9 : 1) ergibt 1.98 g (77 %) reines 6 als farblosen nichtkristallinen Feststoff. IR-Spektrum in $CH_2Cl_2$: 2980, 1770, 1755, 1715, 1610, 1525, 1350, 1245, 1190 $cm^{-1}$.

Weitere Umsetzungen:

Nach den Vorschriften für die Herstellung von (5R,6R)-trans-2-tert-Butyl-6-((1S)-hydroxyethyl)oxapenem-3-carbonsäure (Beispiel 1) werden ausgehend von racemischem 6 ($R^1$, $R^3$, $R^4$, $R^5$ = $CH_3$) über die Stufen Chlorierung, Cyclisierung und katalytische Hydrierung die racemischen Verbindungen 9a (trans) und 9b (cis) erhalten (als Kaliumsalze). Verbindung 9b enthält ca. 20 % 9a.

9a: NMR-Spektrum in $D_2O$: $\delta$ = 1.25 (s,9H), 1.34 (d, 3H, J = 6 Hz), 3.84 (d, 1H, J = 4.5 Hz) 4.27 bis 4.30 (m, 1H), 5.73 (s, 1H).

9b: NMR-Spektrum in $D_2O$: $\delta$ = 1.26 (s, 9H), 1.34 (d, 3H, J = 6.4 Hz), 3.88 bis 3.90 (dd, 1H, J = 3.2 Hz, J = 8.8 Hz) 4.26 bis 4.30 (m, 1H) 5.79 (d, 1H, J = 3.2 Hz).

Beispiel 3

Alternative Herstellung von (5R,6R)-trans-2-tert-butyl-6-((1'S)-hydroxyethyl)oxapenem-3-carbonsäure-Kaliumsalz ohne Konfigurationsumkehr

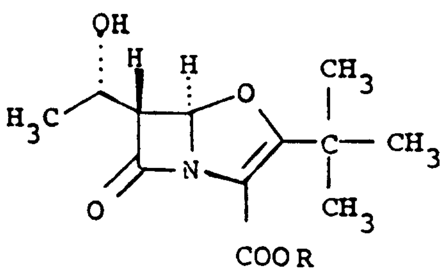

R = H, K

Substitution: Zwischenprodukt 11 ($R^1$ = $CH_3$)

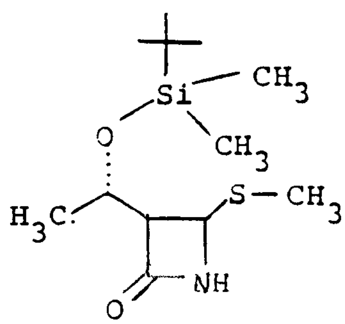

In einem Dreihalskolben mit Rührer und Tropftrichter wird zu einer Suspension von (3R,4R)-4-Acetoxy-3-((1S)-tert-butyldimethylsilyloxyethyl)azetidin-2-on (28.74 g) in 110 ml Acetonitril bei 0°C unter heftigem Rühren eine Lösung von Natriummethanthiolat (10.52 g) in 110 ml Wasser innerhalb von 15 Minuten zugetropft. Das farblose Reaktionsgemisch wird anschliessend bei Raumtemperatur während 75 Minuten gerührt. Das Gemisch wird mit 500 ml Methylenchlorid verdünnt und mit 250 ml Wasser gewaschen. Die wässrige Phase wird noch mit drei Portionen von je 200 ml Methylenchlorid extrahiert und die vereinigten organischen Phasen über Magnesium getrocknet. Eindampfen der Lösung im Vakuum ergibt 11 als nichtkristallinen Festkörper. Ausbeute 23.9 g (87 %). IR-Spektrum in $CH_2Cl_2$: 3400, 2960, 2930, 2860, 1775, 1365, 1145, 1070 $cm^{-1}$.

Alkylierung: Zwischenprodukt 12 ($R^1 = CH_3$)

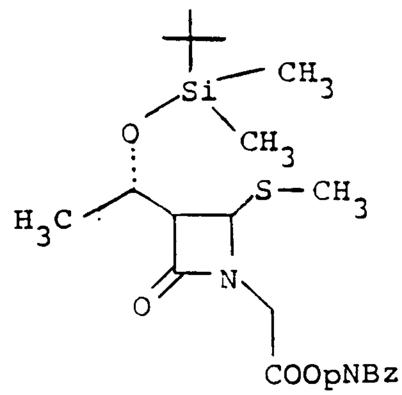

In einem Vierhalskolben mit Rührer, Gummiseptum und Stickstoff-Ballon wird bei -70°C eine Lösung von Zwischenprodukt 11 ($R^1 = CH_3$) (24.8 g) in 196 ml trockenem Tetrahydrofuran langsam mit 36 ml einer 2.5 M Lösung von Butyllithium in Hexan versetzt. Die erhaltene Lösung wird noch während 20 Minuten bei -20°C gerührt und diese Lösung mit einer Spritze zu einer Lösung von Iodessigsäure-p-nitrobenzylester (34.7 g) in 106 ml trockenem N,N-Dimethylformamid gegeben innerhalb von 5 Minuten und unter heftigem Rühren. Das Reaktionsgemisch wird noch während 50 Minuten bei 0°C gerührt und dann mit 2 lt Ether verdünnt, und mit zwei Portionen von je 400 ml 2 %-iger NaCl-Lösung gewaschen. Die vereinigten wässrigen Phasen werden nochmals mit 300 ml Ether extrahiert und die vereinigten organischen Extrakte über Magnesiumsulfat getrocknet. Eindampfen der Lösung im Vakuum ergibt das Rohprodukt 12. Chromatographie auf 1.6 kg Kieselgel mit Toluol-Ethylacetat (97 : 3) und Toluol-Ethylacetat (9 : 1) ergibt 31.7 g (75 %) 12 als hochviskoses, farbloses Öl. IR-Spektrum in $CH_2Cl_2$: 3050, 2955, 2925, 2855, 1760, 1610, 1530, 1380, 1350, 1185 $cm^{-1}$.

Acylierung: Zwischenprodukt 13 ($R^1$, $R^3$, $R^4$, $R^5 = CH_3$)

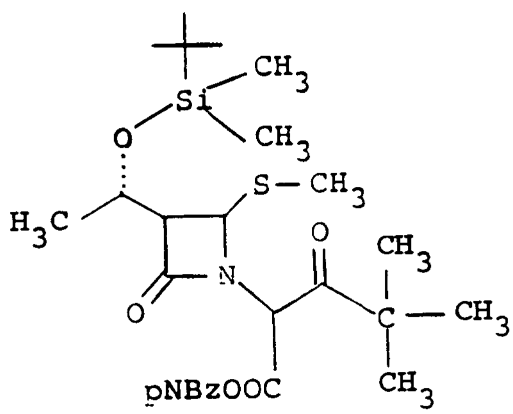

Nach dem für die Herstellung von 2 ($R^1$, $R^3$, $R^4$, $R^5 = CH_3$) angegebenen Verfahren wird ausgehend von 12 ($R^1$, $R^3$, $R^4$, $R^5 = CH_3$) das Zwischenprodukt 13 als nichtkristallines viskoses Öl in 90 % Ausbeute erhalten. IR-Spektrum in $CH_2Cl_2$: 2920, 2850, 1760, 1750, 1605, 1350 $cm^{-1}$.

Deprotection: Zwischenprodukt 5 ($R^1$, $R^3$, $R^4$, $R^5$ = $CH_3$)

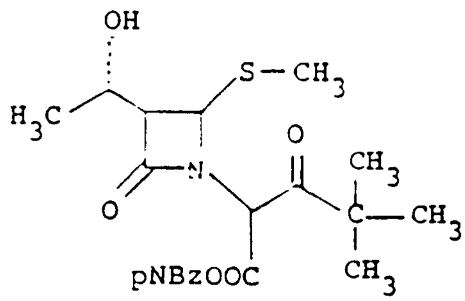

Nach dem für die Herstellung von 3 ($R^1$, $R^3$, $R^4$, $R^5$ = $CH_3$) beschriebenen Verfahren (Beispiel 1) wird ausgehend von 13 das Zwischenprodukt 5 als farbloser nichtkristalliner Feststoff in 80 % Ausbeute erhalten. IR-Spektrum in $CH_2Cl_2$: 3600, 2970, 1765, 1755, 1715, 1610, 1525, 1350 $cm^{-1}$.

Beispiel 4

Umwandlung der Hydroxyalkylseitenkette: (5R,6R)-2-tert-Butyl-6-((1'S)-sulfoxyethyl)oxapenem-3-carbonsäure-dinatrium salz ($R^1$, $R^3$, $R^4$, $R^5$ = $CH_3$; $R^2$ = $SO_3H$)

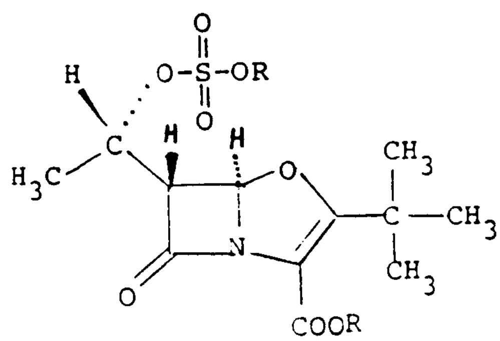

R = H, Na

Zu einer Suspension von (5R,6R)-2-tert-Butyl-6-((1S)-hydroxyethyl)oxapenem-3-carbonsäure-Kaliumsalz (14 mg) in 3 ml trockenm Tetrahydrofuran wird 20 mcl Chlorsulfonsäure-trimethylsilylester unter Rühren bei 0°C gegeben und das Reaktionsgemisch noch 15 Minuten bei 0°C gerührt. Das Reaktionsgemisch wird anschliessend mit 2.4 ml vorgekühltem Phosphatpuffer pH = 7.4 versetzt und während 30 Minuten gerührt. Chromatographie auf reverse-phase-Kieselgel (RP-18) in Acetonitril-Wasser (1 : 4) bei 0°C ergibt 7.5 mg reines Sulfat als Dinatriumsalz. UV-Spektrum in $H_2O$: $\lambda_{max}$ = 275 nm ($\varepsilon$ = 5000). NMR-Spektrum in $D_2O$: 1.26 (s, 9H), 1.48 (d, J = 6.5 Hz, 3H), 4.04 (d, J=3.5Hz, 1H), 4.89 (m, 1H), 5.84 (s, 1H).

Beispiel 5

Herstellung pharmazeutischer Zubereitungen

Eine Einheitsdosisform wird hergestellt, indem man 60 mg (5R,6R)-tert-Butyl-6-((1'S)-(hydroxyethyl)oxapenem-3-carbonsäure, K-Salz mit 120 mg Ampicillin, 20 mg Lactose und 5 mg Magnesiumstearat vermischt und das 205 mg Gemisch in eine Nr. 3 Gelatinekapsel gibt. Ähnlich kann man, wenn man mehr aktive Bestandteile und weniger Lactose verwendet, andere Dosisformen herstellen und in die Nr. 3 Gelatinekapseln füllen; und sollte es erforderlich sein, mehr als 205 mg Bestandteile zusammen zu vermischen, können grössere Kapseln, wie auch komprimierte Tabletten und Pillen, ebenfalls hergestellt werden. Die folgenden Beispiele erläutern die Herstellung pharmazeutischer Zubereitungen.

Tablette

| | mg |
|---|---|
| (5R,6R)-tert-Butyl-6-((1'S)-hydroxyethyl)oxapenem-3-carbonsäure, K-Salz | 60 |
| Ampicillin | 120 |
| Maisstärke V.S.P. | 6 |
| Magnesiumstearat | 232 |
| Dicalciumphosphat | 192 |
| Lactose V.S.P. | 190 |

Die aktiven Bestandteile werden mit dem Calciumphosphat, Lactose und etwa der Hälfte der Maisstärke vermischt. Das Gemisch wird dann mit 6 mg Maisstärke granuliert und grob gesiebt. Es wird im Hochvakuum getrocknet und erneut durch Siebe mit lichten Maschenweiten von 1.00 mm (Nr. 16 screens) gesiebt. Der Rest der Maisstärke und das Magnesiumstearat werden zugegeben und das Gemisch wird zu Tabletten, die je 800 mg wiegen und einen Durchmesser von etwa 1.27 cm (0.5 in.) besitzen, verpresst.

Parenterale Lösung

| | |
|---|---|
| (5R,6R)-tert-Butyl-6-((1'S)-hydroxyethyl)oxapenem-3-carbonsäure, K-Salz | 100 mg |
| Ampicillin | 500 mg |
| steriles Wasser (wird aus einer separaten Ampulle mit der Spritze unmittelbar vor der Verwendung zugegeben) | 2 ml |

Ophthalmische Lösung

| | |
|---|---|
| (5R,6R)-2-tert-Butyl-6-((1'S)-hydroxyethyl)oxapenem-3-carbonsäure, K-Salz | 20 mg |
| Ampicillin | 100 mg |
| Hydroxypropylmethylcellulose | 5 mg |
| steriles Wasser bis zu (wird aus einer separaten Ampulle mit der Spritze unmittelbar vor der Verwendung zugegeben) | 1 ml |

Otische Lösung

| | |
|---|---|
| (5R,6R)-2-tert-Butyl-6-((1'S)-hydroxyethyl)oxapenem-3-carbonsäure, K-Salz | 20 mg |
| Ampicillin | 100 mg |
| Benzalkoniumchlorid | 0.1 mg |
| steriles Wasser bis zu (wird mit der Spritze unmittelbar vor der Verwendung aus einer separaten Ampulle zugegeben) | 1 ml |

Topische Creme bzw. Salbe

| | |
|---|---|
| (5R,6R)-2-tert-Butyl-6-((1'S)-hydroxyethyl)oxapenem-3-carbonsäure, K-Salz | 20 mg |
| Ampicillin | 100 mg |
| Polyethylenglykol 4000 V.S.P. | 400 mg |
| Polyethylenglykol 400 V.S.P. | 100 mg |

**Patentansprüche**

1. Verbindungen der Strukturformel

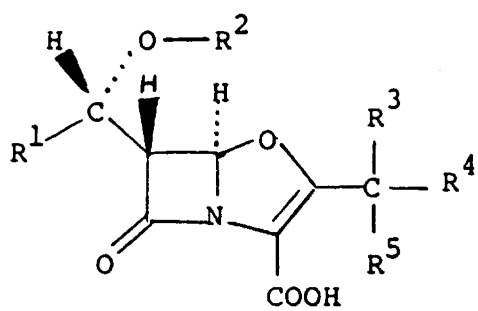

ihre pharmazeutisch annehmbaren Salze, Ester und Amidderivate, worin $R^1$, $R^3$, $R^4$ und $R^5$ unabhängig voneinander pharmazeutisch annehmbare Gruppen mit 1 bis 10 Kohlenstoffatomen bedeuten, die via Kohlenstoff-Kohlenstoff-Einfachbindungen mit dem übrigen Molekülteil verbunden sind, die ausgewählt werden aus den Gruppen: substituiertes oder unsubstituiertes Alkyl, Alkenyl, Alkinyl, Cycloalkyl, Alkylcycloalkyl, Alkylcycloalkenyl Cycloalkylalkyl, Alkenylcycloalkyl, Cycloalkenylalkyl, Aryl, Aralkyl, Aralkenyl, Aralkinyl, Carboxy oder Cyano, worin die vorhergehenden Alkyl-, Alkenyl- oder Alkinyl-Molekülteile 1 bis 6 Kohlenstoffatome, die Cycloalkyl- oder die Cycloalkenyl-Molekülteile 3 bis 6 und die Aryl-Molekülteile 6 bis 10 Kohlenstoffatome enthalten, aromatisches oder aliphatisches Heterocyclyl, Heterocyclylalkyl, Heterocyclylalkenyl, Heterocyclylalkinyl, Alkylheterocyclyl, worin die vorhergehenden Alkyl-, Alkenyl- oder die Alkinyl-Molekülteile 1 bis 6 Kohlenstoffatome enthalten und der heterocycliche Molekülteil mono- oder bicyclisch ist und 3 bis 10 Ringatome enthält, wovon eines oder mehrere ausgewählt werden aus der Gruppe: Sauerstoff, Schwefel und Stickstoff, und wobei die Substituenten der ober aufgeführten Gruppen sein können: Geschütztes oder ungeschütztes Hydroxy, Hydroxyalkyl, Aninoalkyloxy, Alkyloxy, Acyloxy, Aryloxy, Heterocyclyloxy, Carbamoyl, Carbamoyloxy, Thiocarbamoyl, Thiocarbamoyloxy, Alkylcarbamoyloxy, Alkylthiocarbamoyloxy, Mercapto, Alkylthio, Hydroxyalkylthio, Aminoalkylthio, Amidinoalkylthio, Acylthio, Arylthio, Alkylheteroarylthio, Hydroxyalkylheteroarylthio, Heterocyclylthio, Carbamoylthio, Alkylcarbamoylthio, Thiocarbamoylthio, Alkylthiocarbamoylthio, geschütztes oder ungeschütztes Amino oder Monoalkylamino, Dialkylamino, Oxo, geschütztes oder ungeschütztes Oximino oder Alkylimino, Tetraalkylammonium, Cycloalkylamino, Arylamino, Heteroarylamino, Heterocyclylamino, Acylamino, Amidino, Alkylamidino, Guanidino, Alkylguanidino, Carbamoylamino, Alkylcarbamoylamino, Thiocarbamoylamino, Alkylthiocarbamoylamino, Nitro, chlor, Brom, Fluor, Iod, Azido, Cyano, Alkylsulfinyl, Alkylsulfonyl, Sulfonamido, Sulfamoyloxy, Alkylsulfonyloxy, oder geschütztes oder ungeschütztes Sulfo, Sulfoxy oder Carboxy, wobei die Substituenten unabhängig voneinander ein- oder mehrfach auftreten und ihr Alkyl-Molekülteil 1 bis 6 Kohlenstoffatome, ihr Aryl-Molekülteil 6 bis 10 Kohlenstoffatome enthält, und wobei der heterocyclische Molekülteil mono- oder bicyclisch ist und 3 bis 10 Ringatome enthält, wovon eines oder mehrere ausgewählt werden aus der Gruppe: Sauerstoff, Schwefel und Stickstoff, und in der $R^2$ Wasserstoff, Alkyl- oder Acyl mit 1 bis 10 Kohlenstoffatomen, Sulfo, Aminosulfonyl oder Phosphoroyl bedeuten.

**2.** Verbindungen nach Anspruch 1, dadurch gekennzeichnet, dass $R^1$ ausgewählt wird aus den Gruppen:

$CH_3$ $CH_2$-$CH_3$ $H_2C$=CH $H_2C$=CH-$CH_2$ HC≡C HC≡C-$CH_2$

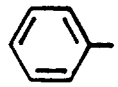
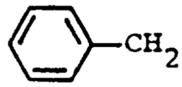
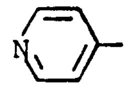
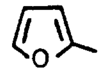

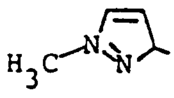
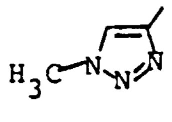
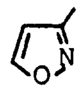
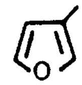

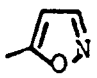
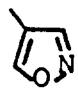
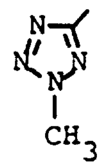
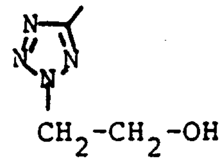

und $R^2$ Wasserstoff, Alkyl oder Acyl mit 1 bis 10 Kohlenstoffatomen, Sulfo, Aminosulfonyl oder Phosphoroyl bedeutet, $R^3$ und $R^4$ Methyl bedeuten und $R^5$ ausgewählt wird aus den Gruppen:

$CH_3$ $CH_2\text{-}CH_3$ $H_2C{=}CH$ $H_2C{=}CH\text{-}CH_2$ $HC{\equiv}C$ $HC{\equiv}C\text{-}CH_2$

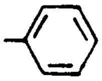 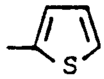 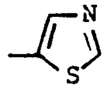

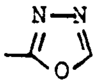 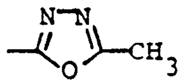 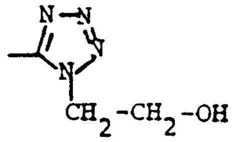

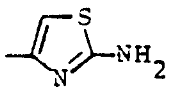 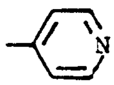 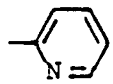

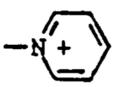 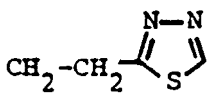 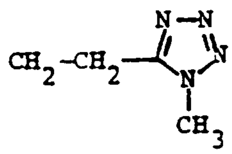

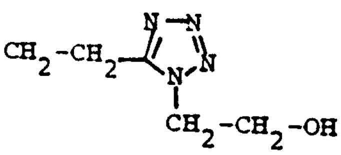 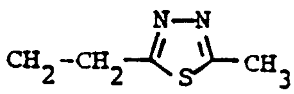

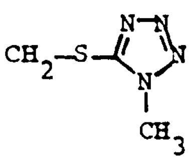 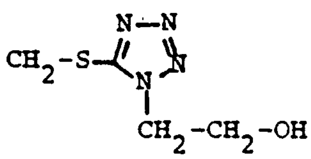 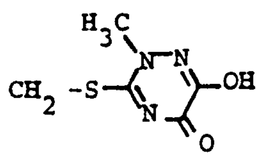

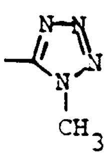 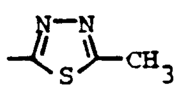 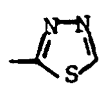

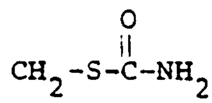

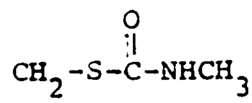

$CH_2$-S-$CH_3$ $CH_2$-S-$CH_2$-$CH_2$-$NH_2$

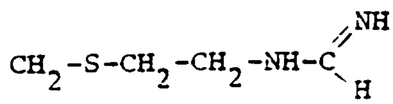

$CH_2$-$NH_2$

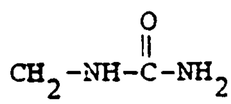

$CH_2$-NH-CHO

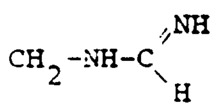

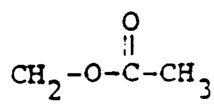

$CH_2$-O-CHO $CH_2$-Cl

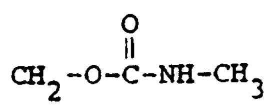

$CH_2$-$N_3$

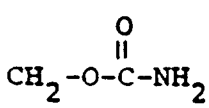

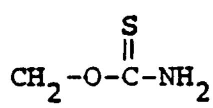

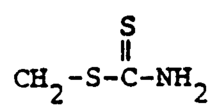

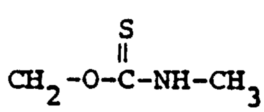

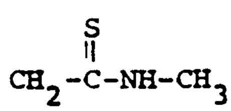

$CH_2$-$CH_2$-$NH_2$

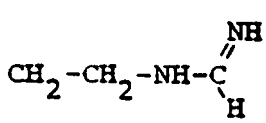

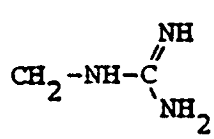

$CH_2$-$CH_2$-$CH_2$-$NH_2$

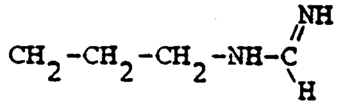

$CH_2$-$CH_2$-COOH $CH_2$-COOH $CH_2$-O-$CH_3$

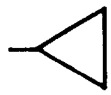

COOH $CH_2$-OH $CH_2$-$CH_2$-OH

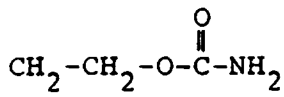

C≡N

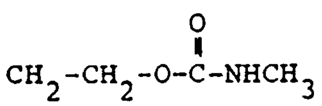

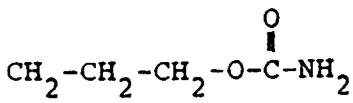

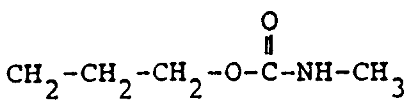

$CH_2$-$CH_2$-NH-CHO

**3.** Verbindungen nach Anspruch 2, dadurch gekennzeichnet, dass $R^1$ und $R^2$ die oben angegebenen Bedeutungen haben und $R^3$, $R^4$ und $R^5$ Methyl bedeuten.

**4.** Verbindungen nach Anspruch 3, dadurch gekennzeichnet, dass $R^2$ Wasserstoff bedeutet.

**5.** Pharmazeutische Zubereitung, dadurch gekennzeichnet, dass sie eine Verbindung nach mindestens einem der Ansprüche 1 bis 4 und einen pharmazeutischen Träger dafür enthält.

**6.** Pharmazeutische Zubereitung, dadurch gekennzeichnet, dass sie in Dosiseinheitsform vorliegt und eine therapeutisch wirksame Menge einer Verbindung nach mindestens einem der Ansprüche 1 bis 4 und einen pharmazeutischen Träger dafür enthält.

**7.** Pharmazeutische Zubereitung, dadurch gekennzeichnet, dass sie eine Verbindung nach mindestens einem der Ansprüche 1 bis 4, ein Betalactamantibiotikum und einen pharmazeutischen Träger dafür enthält.

**8.** Pharmazeutische Zubereitung, dadurch gekennzeichnet, dass sie in Dosiseinheitsform vorliegt, eine therapeutisch wirksame Menge einer Verbindung nach mindestens einem der Ansprüche 1 bis 4, ein Betalactamantibiotikum und einen pharmazeutischen Träger dafür enthält.

**9.** Verwendung von Verbindungen nach Anspruch 1 zur Herstellung betalactamasehemmender Arzneimittel.

**10.** Verfahren zur Herstellung von Verbindungen nach Anspruch 1, dadurch gekennzeichnet, dass optisch aktives, geschütztes oder ungeschütztes 4-Acetoxy-3-((1'S)-hydroxyalkyl)azetidin-2-on als Ausgangs- oder Zwischenprodukt verwendet wird.

11. Verfahren zur Herstellung von Verbindungen nach Anspruch 1, dadurch gekennzeichnet, dass optisch aktives, geschütztes oder ungeschütztes 4-Acetoxy-3-((1'R)-hydroxyalkyl)azetidin-2-on als Ausgangsmaterial verwendet wird.

12. Racemische 6-(1'Hydroxyalkyl)-oxapenem-3-carbonsäuren nach mindestens einem der Ansprüche 1 - 4.

13. Verwendung von Verbindungen nach Anspruch 12 zur Herstellung belactamasehemmender Arzneimittel.

**Claims**

1. Compounds of the structural formula

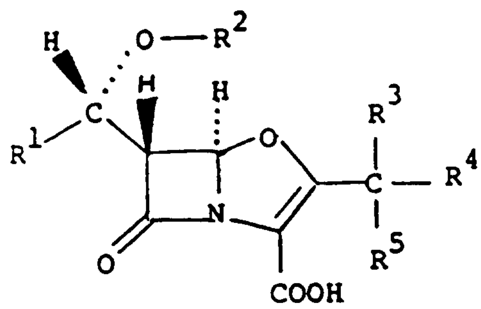

their pharmaceutically acceptable salts, esters and amide derivatives, in which $R^1$, $R^3$, $R^4$ and $R^5$ independently of one another are pharmaceutically acceptable groups having 1 to 10 carbon atoms, which are bonded to the other entity via carbon-carbon single bonds and which are selected from the groups:
substituted or unsubstituted alkyl, alkenyl, alkynyl, cycloalkyl, alkylcycloalkyl, alkylcycloalkenyl, cycloalkylalkyl, alkenylcycloalkyl, cycloalkenylalkyl, aryl, aralkyl, aralkenyl, aralkynyl, carboxyl or cyano, in which the above alkyl, alkenyl or alkynyl entities contain 1 to 6 carbon atoms, the cycloalkyl or the cycloalkenyl entities 3 to 6 and the aryl entities 6 to 10 carbon atoms, aromatic or aliphatic heterocyclyl, heterocyclylalkyl, heterocyclylalkenyl, heterocyclylalkynyl, alkylheterocyclyl, in which the above alkyl, alkenyl or the alkynyl entities contain 1 to 6 carbon atoms and the heterocyclic entity is mono- or bicyclic and contains 3 to 10 ring atoms, of which one or more are selected from the group consisting of: oxygen, sulphur and nitrogen, and where the substituents can be of the abovementioned groups: protected or unprotected hydroxyl, hydroxyalkyl, aminoalkoxy, alkoxy, acyloxy, aryloxy, heterocyclyloxy, carbamoyl, carbamoyloxy, thiocarbamoyl, thiocarbamoyloxy, alkylcarbamoyloxy, alkylthiocarbamoyloxy, mercapto, alkylthio, hydroxyalkylthio, aminoalkylthio, amidinoalkylthio, acylthio, arylthio, alkylheteroarylthio, hydroxyalkylheteroarylthio, heterocyclylthio, carbamoylthio, alkylcarbamoylthio, thiocarbamoylthio, alkylthiocarbamoylthio, protected or unprotected amino or monoalkylamino, dialkylamino, oxo, protected or unprotected oximino or alkylimino, tetraalkylammonium, cycloalkylamino, arylamino, heteroarylamino, heterocyclylamino, acylamino, amidino, alkylamidino, guanidino, alkylguanidino, carbamoylamino, alkylcarbamoylamino, thiocarbamoylamino, alkylthiocarbamoylamino, nitro, chlorine, bromine, fluorine, iodine, azido, cyano, alkylsulphinyl, alkylsulphonyl, sulphonamido, sulphamoyloxy, alkylsulphonyloxy, or protected or unprotected sulpho, sulphoxyl or carboxyl, where the substituents independently of one another occur one or more times and their alkyl entity contains 1 to 6 carbon atoms and their aryl entity 6 to 10 carbon atoms, and where the heterocyclic entity is mono- or bicyclic and contains 3 to 10 ring atoms, of which one or more are selected from the group consisting of: oxygen, sulphur and nitrogen, and in which $R^2$ is hydrogen, alkyl or acyl having 1 to 10 carbon atoms, sulpho, aminosulphonyl or phosphoroyl.

2. Compounds according to Claim 1, characterized in that $R^1$ is selected from the groups:
$CH_3$ $CH_2\text{-}CH_3$ $H_2C{=}CH$ $H_2C{=}CH\text{-}CH_2$ $HC{\equiv}C$ $HC{\equiv}C\text{-}CH_2$

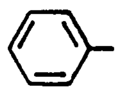 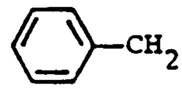 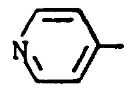 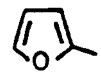

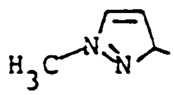 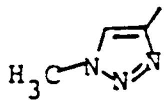 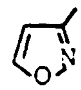 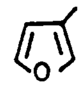

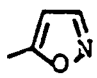 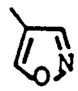 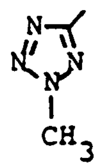 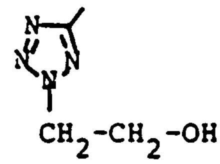

and $R^2$ is hydrogen, alkyl or acyl having 1 to 10 carbon atoms, sulpho, aminosulphonyl or phosphoroyl, $R^3$ and $R^4$ are methyl and $R^5$ is selected from the groups:
$CH_3$ $CH_2\text{-}CH_3$ $H_2C{=}CH$ $H_2C{=}CH\text{-}CH_2$ $HC{\equiv}C$ $HC{\equiv}C\text{-}CH_2$

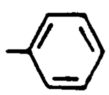 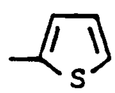 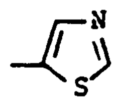

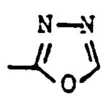 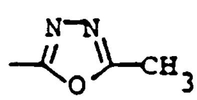 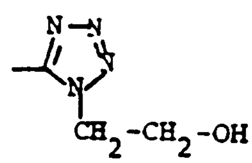

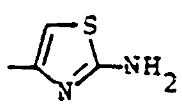 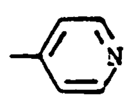 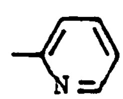

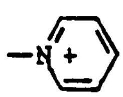 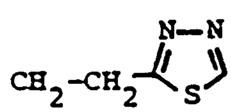 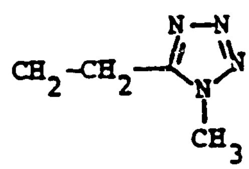

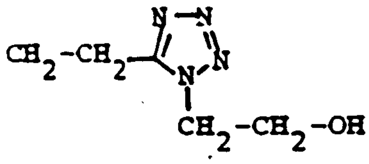

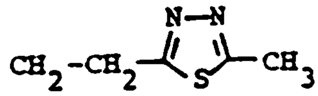

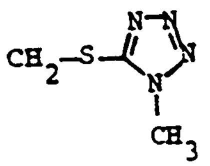

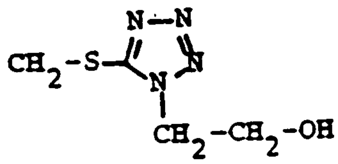

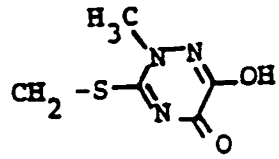

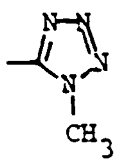

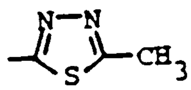

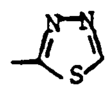

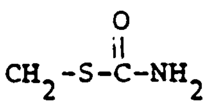

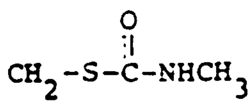

$CH_2$-S-$CH_3$ $CH_2$-S-$CH_2$-$CH_2$-$NH_2$

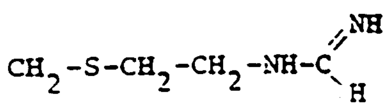

$CH_2$-$NH_2$

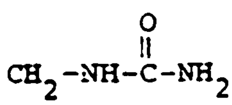

$CH_2$-NH-CHO

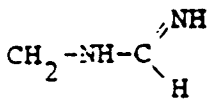

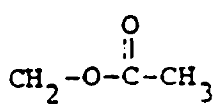

$CH_2$-O-CHO $CH_2$-Cl

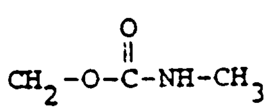

$CH_2\text{-}N_3$

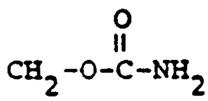 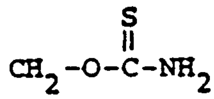 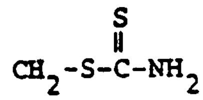

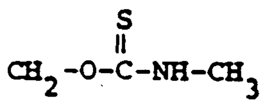 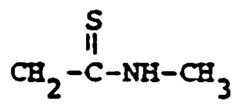

$CH_2\text{-}CH_2\text{-}NH_2$

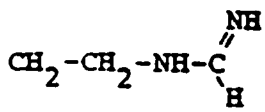 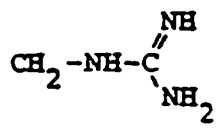

$CH_2\text{-}CH_2\text{-}CH_2\text{-}NH_2$

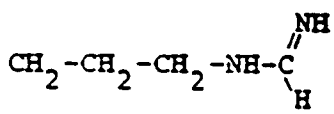

$CH_2\text{-}CH_2\text{-}COOH$ $CH_2\text{-}COOH$ $CH_2\text{-}O\text{-}CH_3$

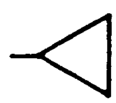

COOH $CH_2\text{-}OH$ $CH_2\text{-}CH_2\text{-}OH$

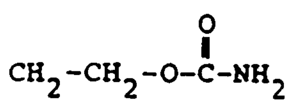

$C{\equiv}N$

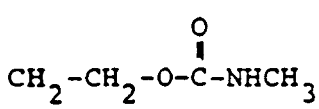 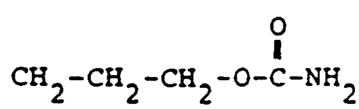

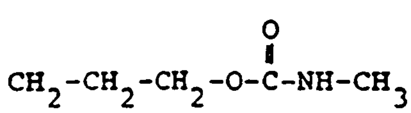

$CH_2\text{-}CH_2\text{-}NH\text{-}CHO$

3. Compounds according to Claim 2, characterized in that $R^1$ and $R^2$ have the meanings indicated above and $R^3$, $R^4$ and $R^5$ are methyl.

4. Compounds according to Claim 3, characterized in that $R^2$ is hydrogen.

5. Pharmaceutical preparation, characterized in that it contains a compound according to at least one of Claims 1 to 4 and a pharmaceutical excipient therefor.

6. Pharmaceutical preparation, characterized in that it is present in dose unit form and contains a therapeutically active amount of a compound according to at least one of Claims 1 to 4 and a pharmaceutical excipient therefor.

7. Pharmaceutical preparation, characterized in that it contains a compound according to at least one of Claims 1 to 4, a beta-lactam antibiotic and a pharmaceutical excipient therefor.

8. Pharmaceutical preparation, characterized in that it is present in dose unit form and contains a therapeutically active amount of a compound according to at least one of Claims 1 to 4, a beta-lactam antibiotic and a pharmaceutical excipient therefor.

9. Use of compounds according to Claim 1 for the production of beta-lactamase-inhibiting medicaments.

10. Process for the production of compounds according to Claim 1, characterized in that optically active, protected or unprotected 4-acetoxy-3-((1'S)-hydroxyalkyl)azetidin-2-one is used as a starting material or intermediate.

11. Process for the preparation of compounds according to Claim 1, characterized in that optically active, protected or unprotected 4-acetoxy-3-((1'R)-hydroxyalkyl)azetidin-2-one is used as a starting material.

12. Racemic 6-(1'-hydroxyalkyl)oxapenem-3-carboxylic acids according to at least one of Claims 1 - 4.

13. Use of compounds according to Claim 12 for the production of beta-lactamase-inhibiting medicaments.

**Revendications**

1. Composés répondant à la formule développée :

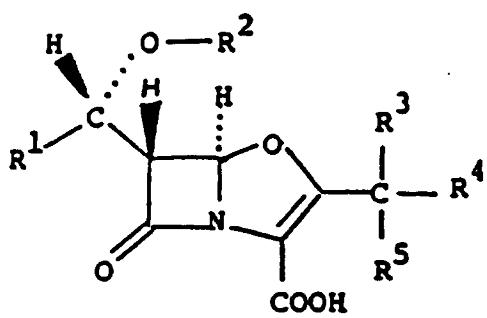

leurs sels, esters et dérives d'amides pharmaceutiquement acceptables, dans laquelle $R^1$, $R^3$, $R^4$ et $R^5$ représentent, indépendamment l'un de l'autre, des groupes pharmaceutiquement acceptables contenant de 1 à 10 atomes de carbone, qui sont reliés à la partie restante de la molécule via des liaisons simples carbone-carbone, qui sont choisis parmi les groupes ci-après : un groupe alkyle, un groupe alcényle, un groupe alcynyle, un groupe cycloalkyle, un groupe alkylcycloalkyle, un groupe alkylcycloalcényle, un groupe cycloalkylalkyle, un groupe alcénylcycloalkyle, un groupe cycloalcénylalkyle, un groupe aryle, un groupe aralkyle, un groupe aralcényle, un groupe aralcynyle, un groupe carboxyle ou un groupe cyano, chacun de ces groupes étant substitué ou non substitué, les fractions moléculaires alkyle, alcényle ou alcynyle précitées contenant de 1 à 6 atomes de carbone, les fractions moléculaires cycloalkyle ou cycloalcényle contenant de 3 à 6 atomes de carbone et les fractions moléculaires aryle contenant de 6 à 10 atomes de carbone; un groupe hétérocyclyle, un groupe hétérocyclylalkyle, un groupe hétérocyclylalcényle, un groupe hétérocyclylalcynyle, un groupe alkylhétérocyclyle, chacun de ces groupes étant aromatiques ou aliphatiques, les fractions moléculaires alkyle, alcényle ou alcynyle précitées contenant de 1 à 6 atomes de carbone et la fraction moléculaire hétérocyclique étant de type mono- ou bicyclique et contenant de 3 à 10 atomes cycliques

dont un ou plusieurs sont choisis parmi le groupe comprenant l'oxygène, le soufre et l'azote, les substituants des groupes indiqués ci-dessus pouvant être : un groupe hydroxyle, un groupe hydroxyalkyle, un groupe aminoalcoxy, un groupe alcoxy, un groupe acyloxy, un groupe aryloxy, un groupe hétérocyclyloxy, un groupe carbamoyle, un groupe carbamoyloxy, un groupe thiocarbamoyle, un groupe thiocarbamoyloxy, un groupe alkylcarbamoyloxy, un groupe alkylthiocarbamoyloxy, un groupe mercapto, un groupe alkylthio, un groupe hydroxyalkylthio, un groupe aminoalkylthio, un groupe amidinoalkylthio, un groupe acylthio, un groupe arylthio, un groupe alkylhétéroarylthio, un groupe hydroxyalkyl-hétéroarylthio, un groupe hétérocyclylthio, un groupe carbamoylthio, un groupe alkylcarbamoylthio, un groupe thiocarbamoylthio, un groupe alkylthiocarbamoylthio, chacun de ces substituants étant protégé ou non; un groupe amino ou un groupe monoalkylamino protégé ou non protégé; un groupe dialkylamino, un groupe oxo; un groupe oximino ou un groupe alkylimino protégé ou non protégé; un groupe tétraalkylammonium, un groupe cycloalkylamino, un groupe arylamino, un groupe hétéroarylamino, un groupe hétérocyclylamino, un groupe acylamino, un groupe amidino, un groupe alkylamidino, un groupe guanidino, un groupe alkylguanidino, un groupe carbamoylamino, un groupe alkylcarbamoylamino, un groupe thiocarbamoylamino, un groupe alkylthiocarbamoylamino, un groupe nitro, un atome de chlore, un atome de brome, un atome de fluor, un atome d'iode, un groupe azido, un groupe cyano, un groupe alkylsulfinyle, un groupe alkylsulfonyle, un groupe sulfonamido, un groupe sulfamoyloxy, un groupe alkylsulfonyloxy; ou encore un groupe sulfo, un groupe sulfoxy ou un groupe carboxyle, chacun de ces groupes étant protégé ou non, les substituants étant présents, indépendamment l'un de l'autre, une ou plusieurs fois, et leurs fractions moléculaires alkyle contenant de 1 à 6 atomes de carbone, leurs fractions moléculaires aryle contenant de 6 à 10 atomes de carbone, et la fraction moléculaire hétérocyclique étant de type mono- ou bicyclique et contenant de 3 à 10 atomes cycliques dont un ou plusieurs sont choisis parmi le groupe comprenant l'oxygène, le soufre et l'azote, et dans laquelle $R^2$ représente un atome d'hydrogène, un groupe alkyle ou un groupe acyle contenant de 1 à 10 atomes de carbone, un groupe sulfo, un groupe aminosulfonyle ou un groupe phosphoroyle.

2. Composés selon la revendication 1, caractérisés en ce que $R^1$ est choisi parmi les groupes :
$CH_3$ $CH_2$-$CH_3$ $H_2C$=CH $H_2C$=CH-$CH_2$ HC≡C HC≡C-$CH_2$

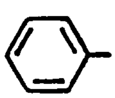 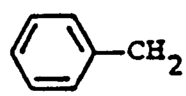 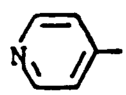 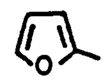

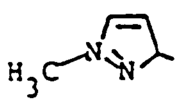 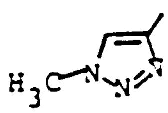 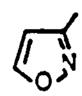 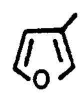

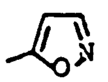 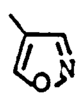 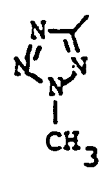 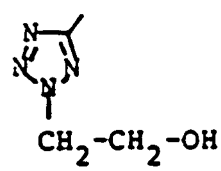

et $R^2$ représente un atome d'hydrogène, un groupe alkyle ou un groupe acyle contenant de 1 à 10 atomes de carbone, un groupe sulfo, un groupe aminosulfonyle ou un groupe phosphoroyle, $R^3$ et $R^4$ représentant un groupe méthyle et $R^5$ étant choisi parmi les groupes :

$CH_3$ $CH_2$-$CH_3$ $H_2C$=CH $H_2C$=CH-$CH_2$ HC≡C HC≡C-$CH_2$

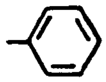 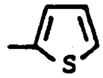 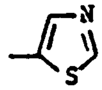

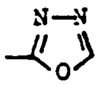 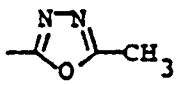 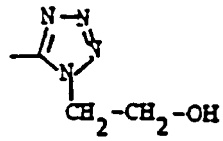

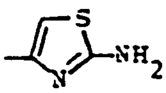 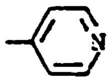 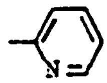

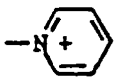 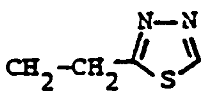 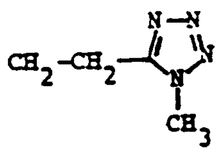

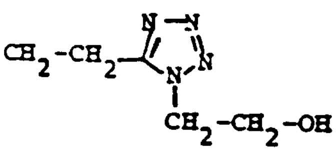 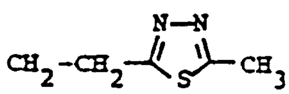

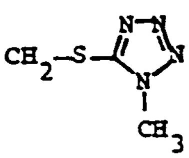 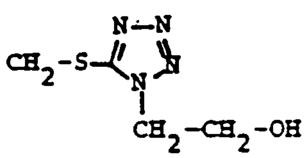 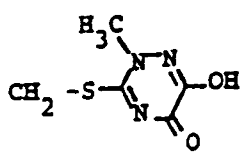

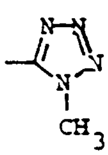 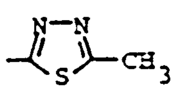 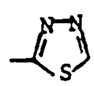

$CH_2$-O-$CH_3$

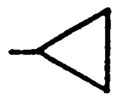

COOH $CH_2$-OH $CH_2$-$CH_2$-OH

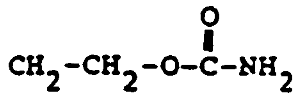

C≡N

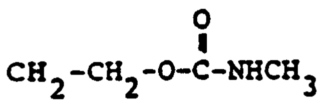

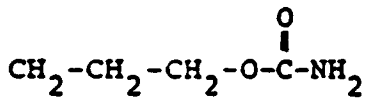

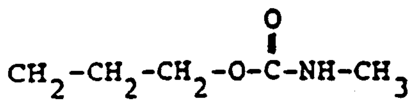

$CH_2$-$CH_2$-NH-CHO

3. Composés selon la revendication 2, caractérisés en ce que $R^1$ et $R^2$ ont les significations indiquées ci-dessus et $R^3$, $R^4$ et $R^5$ représentent un groupe méthyle.

4. Composés selon la revendication 3, caractérisés en ce que $R^2$ représente un atome d'hydrogène.

5. Préparation pharmaceutique caractérisée en ce qu'elle contient un composé selon au moins une des revendications 1 à 4, ainsi qu'un support pharmaceutique pour le composé.

6. Préparation pharmaceutique caractérisée en ce qu'elle est présente sous forme d'une unité posologique et elle contient une quantité thérapeutiquement efficace d'un composé selon au moins une des revendications 1 à 4, ainsi qu'un support pharmaceutique pour le composé.

7. Préparation pharmaceutique caractérisée en ce qu'elle contient un composé selon au moins une des revendications 1 à 4, un antibiotique β-lactame et un support pharmaceutique pour ce dernier.

8. Préparation pharmaceutique caractérisée en ce qu'elle est présente sous forme d'une unité posologique, elle contient une quantité thérapeutiquement efficace d'un composé selon au moins une des revendications 1 à 4, un antibiotique β-lactame et un support pharmaceutique pour ce dernier.

9. Utilisation de composés selon la revendication 1, pour la préparation de médicaments inhibiteurs de la β-lactamase.

10. Procédé pour la préparation de composés selon la revendication 1, caractérisé en ce qu'on utilise un produit de départ ou un produit intermédiaire optiquement actif, protégé ou non, de 4-acétoxy-3-((1'S)-hydroxyalkyl)azétidin-2-one.

11. Procédé pour la préparation de composés selon la revendication 1, caractérisé en ce qu'on utilise comme matière de départ, la 4-acétoxy-3-((1'R)-hydroxyalkyl)azétidin-2-one optiquement active, protégée ou non.

12. Acides 6-(1'-hydroxyalkyl)-oxapénem-3-carboxyliques racémiques selon au moins une des revendications 1 à 4.

13. Utilisation de composés selon la revendication 12, pour la préparation de médicaments inhibiteurs de la β-lactamase.